(19) Europäisches Patentamt
European Patent Office
Office européen des brevets

(11) EP 3 224 235 B1

(12) **EUROPÄISCHE PATENTSCHRIFT**

(45) Veröffentlichungstag und Bekanntmachung des Hinweises auf die Patenterteilung:
**14.11.2018 Patentblatt 2018/46**

(21) Anmeldenummer: **15798024.4**

(22) Anmeldetag: **18.11.2015**

(51) Int Cl.:
***C07C 209/86*** (2006.01) ***C07C 211/36*** (2006.01)

(86) Internationale Anmeldenummer:
**PCT/EP2015/076968**

(87) Internationale Veröffentlichungsnummer:
**WO 2016/083210 (02.06.2016 Gazette 2016/22)**

# (54) VERFAHREN ZUR HERSTELLUNG VON CIS- UND TRANS-ANGEREICHERTEM MDACH

PROCESS FOR THE PREPARATION OF CIS- AND TRANS-ENRICHED MDACH

PROCÉDÉ DE PRÉPARATION DE CIS- ET TRANS-ENRICHI MDACH

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **25.11.2014 EP 14194717**

(43) Veröffentlichungstag der Anmeldung:
**04.10.2017 Patentblatt 2017/40**

(73) Patentinhaber: **BASF SE**
**67056 Ludwigshafen am Rhein (DE)**

(72) Erfinder:
- **WEIDERT, Jan-Oliver**
  **67105 Schifferstadt (DE)**
- **KRAMP, Sandra**
  **68163 Mannheim (DE)**
- **BENFER, Regina**
  **67122 Altrip (DE)**
- **PANCHENKO, Alexander**
  **67071 Ludwigshafen (DE)**
- **WEICKGENANNT, Andreas**
  **68309 Mannheim (DE)**
- **GUTFRUCHT, Norbert**
  **67466 Lambrecht (DE)**
- **BREUER, Klaus**
  **67346 Speyer (DE)**
- **KOZICKI, Artur**
  **67098 Bad Duerkheim (DE)**
- **BUSCH, Ralph**
  **67549 Worms (DE)**

(74) Vertreter: **BASF IP Association**
**BASF SE**
**G-FLP-C006**
**67056 Ludwigshafen (DE)**

(56) Entgegenhaltungen:
**WO-A1-95/35287** **WO-A1-2011/032877**
**DE-A1- 10 128 242**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 3 224 235 B1

## Beschreibung

Der Gegenstand der vorliegenden Erfindung betrifft ein Verfahren zur Herstellung von trans-angereichertem MDACH. Des Weiteren betrifft die Erfindung ein Verfahren zur Herstellung von cis- und trans-angereichertem MDACH. Ebenso betrifft die Erfindung cis- und trans-angereichertes MDACH.

Die destillative Trennung von cis und trans-Isomeren eines Gemischs aus 2,4-Diamino-1-methylcyclohexan (2,4-MDACH) und 2,6-Diamino-1-methylcyclohexan (2,6-MDACH) ist aufgrund der eng beieinander liegenden Siedepunkte nur mit großem Aufwand möglich.

EP 0 796 839 A1 (Bayer AG) beschreibt die kontinuierliche Herstellung eines Gemischs aus Amino-methylcyclohexanen und Diamino-methyl-cyclohexanen durch katalytische Hydrierung von Diamino-toluolen mit Wasserstoff. Weiter wird die destillative Trennung der erhaltenen Amino-methyl-cyclohexane und Diamino-methyl-cyclohexane beschrieben. Ein zusätzlicher Destillationsschritt zur Anreicherung von cis- oder trans-Isomeren wird nicht gelehrt.

WO 2011/033104 A1 (BASF SE) betrifft unter anderem Mischungen enthaltend die 7 Stereoisomere des Diamino-methyl-cyclohexans in ganz speziellen Mengenverhältnissen zueinander. Die Mischungen werden durch Hydrierung von Toluoldiamin hergestellt, wobei das erhaltene Hydrierungsprodukt destillativ gereinigt wird. Ein zusätzlicher Destillationsschritt zur Anreicherung von cis- oder trans-Isomeren wird auch hier nicht gelehrt.

Für die Weiterreaktion von 2,4- und 2,6-MDACH zu entsprechenden Folgeprodukten kann es vorteilhaft sein, Gemische mit einem erhöhten Anteil an trans-Isomeren einzusetzen. Ebenso vorteilhaft kann der Einsatz von Gemischen mit einem erhöhten Anteil an cis-Isomeren sein.

Aufgabe der vorliegenden Erfindung war es daher, Gemische aus 2,4- und 2,6-MDACH mit einem erhöhten Anteil an trans-Isomeren zur Verfügung zu stellen. Des Weiteren sollten entsprechende Gemische mit einem erhöhten Anteil an cis-Isomeren zur Verfügung gestellt werden. Insbesondere sollte ein Verfahren gefunden werden, mithilfe dessen sich solche Gemische herstellen lassen.

Die Aufgabe wird gelöst durch ein Verfahren zur Herstellung von trans-angereichertem MDACH, das dadurch gekennzeichnet ist, dass die Destillation eines MDACH-Ausgangsgemischs in Gegenwart eines Hilfsstoffs durchgeführt wird, wobei trans-angereichertes MDACH abdestilliert wird,
und wobei

- der Hilfsstoff eine organische Verbindung mit
    - ◦ einer molaren Masse von 62 bis 500 g/mol,
    - ◦ einem Siedepunkt, der mindestens 5 °C über dem Siedepunkt von cis,cis-2,6-Diamino-1-methylcyclohexan liegt, wobei sich die Siedepunkte jeweils auf einen Druck von 50 mbar beziehen, und
    - ◦ 2 bis 4 funktionellen Gruppen, bei denen es sich jeweils unabhängig voneinander um eine Alkohol-, primäre, sekundäre, oder tertiäre Aminogruppe handelt, ist,
- das MDACH-Ausgangsgemisch 0 bis 100 Gew.-% 2,4-Diamino-1-methylcyclohexan (2,4-MDACH) und 0 bis 100 Gew.-% 2,6-Diamino-1-methylcyclohexan (2,6-MDACH), bezogen auf die im MDACH-Ausgangsgemisch enthaltende Gesamtmenge an MDACH (= 2,4- und 2,6-MDACH), enthält und
  wobei das MDACH-Ausgangsgemisch sowohl trans- als auch cis-Isomere enthält,
  und
- trans-angereichertes MDACH ein Gemisch ist, das 0 bis 100 Gew.-% 2,4-MDACH und 0 bis 100 Gew.-% 2,6-MDACH, bezogen auf die im Gemisch enthaltende Gesamtmenge an MDACH, enthält, wobei der Anteil an trans-Isomeren im Gemisch, bezogen auf die im Gemisch enthaltene Gesamtmenge an MDACH, höher ist, als der Anteil an trans-Isomeren im MDACH-Ausgangsgemisch, bezogen auf die im MDACH-Ausgangsgemisch enthaltene Menge an MDACH.

Erfindungsgemäß wurde erkannt, dass sich cis- und trans-Isomere des 2,4-MDACH und 2,6-MDACH durch die Gegenwart des erfindungsgemäßen Hilfsstoffs besser voneinander trennen lassen.

Bei dem Hilfsstoff handelt es sich um eine organische Verbindung. Hierunter ist eine molekulare Verbindung zu verstehen, in der Kohlenstoff in Verbindung mit Wasserstoff enthalten ist. Beispielsweise kann es sich um eine aliphatische Verbindung handeln. Der Begriff aliphatische Verbindung bezeichnet eine organische Verbindung ohne aromatisches Ringsystem. Eine aliphatische Verbindung kann sowohl verzweigt als auch unverzweigt sein und beliebige funktionelle Gruppen beziehungsweise Heteroatome enthalten. Der Begriff aliphatische Verbindungen umfasst ferner auch cyclische aliphatische (cycloaliphatische) Verbindungen. In einer cycloaliphatischen Verbindung sind zumindest ein Teil der sie bildenden Atome im Molekül so miteinander verknüpft ist, dass einer oder mehrere Ringe ausgebildet werden.

Ebenso kann es sich aber auch um eine organische Verbindung handeln, die ein aromatisches Ringsystem, insbesondere einen Tolyl- oder Phenylrest, enthält.

Bei einer funktionellen Gruppe im Sinne der vorliegenden Erfindung handelt es sich um eine Alkohol-, primäre, sekundäre, oder tertiäre Aminogruppe.

Damit trans-angereichertes MDACH über Kopf erhalten werden kann, soll der Großteil des Hilfsstoffs während der Destillation im Sumpf verbleiben beziehungsweise, bei kontinuierlicher Reaktionsführung, über Sumpf abgezogen werden. Der Siedepunkt des Hilfsstoffs muss daher mindestens 5 °C über dem Siedepunkt von cis,cis-2,6-Diamino-1-methylcyclohexan liegen, wobei sich die Siedepunkte jeweils auf einen Druck von 50 mbar beziehen. Das heißt, dass sowohl der Siedepunkt des Hilfsstoffs als auch der Siedepunkt des cis,cis-2,6-Diamino-1-methylcyclo-hexans, das von allen Isomeren des 2,4- und des 2,6-MDACH den höchsten Siedepunkt hat, bei 50 mbar bestimmt wird. Bevorzugt ist der Siedepunkt des Hilfsstoffs um 5 bis 100 °C, besonders bevorzugt 10 bis 50 °C und ganz besonders bevorzugt um 20 bis 30 °C höher als der von cis,cis-2,6-Diamino-1-methylcyclohexan. Bei einem entsprechend hohen Siedepunkt des Hilfsstoffs muss der Sumpf während der Destillation stärker erhitzt werden, was die Zersetzung des MDACH begünstigt. Insofern ist die Obergrenze in den bevorzugten Bereichen entsprechend kleiner.

In einer besonders bevorzugten Ausführungsform beziehen sich die oben genannten Siedepunkte nicht auf 50 mbar sondern auf den Kopfdruck, bei dem die Destillation des MDACH-Ausgangsgemischs stattfindet. Soll die Destillation beispielsweise bei einem Kopfdruck von 40 mbar erfolgen, so kann der Fachmann dies beispielsweise mithilfe einer Vakuumpumpe entsprechend einstellen. Der Fachmann wird hier einen Hilfsstoff auswählen, dessen Siedepunkt bei dem besagten Druck von 40 mbar um mindestens 5 °C beziehungsweise um 5 bis 100 °C, 10 bis 50 °C oder 20 bis 30 °C über dem Siedepunkt, dann ebenfalls bei 40 °C bestimmt, von cis,cis-2,6-Diamino-1-methylcyclohexan liegt.

In dieser besonders bevorzugten Ausführungsform hat der Hilfsstoff folglich einen Siedepunkt, der mindestens 5 °C, bevorzugt 5 bis 100 °C, besonders bevorzugt 10 bis 50 °C und ganz besonders bevorzugt 20 bis 30 °C über dem Siedepunkt von cis,cis-2,6-Diamino-1-methylcyclohexan liegt, wobei sich die Siedepunkte jeweils auf den Kopfdruck beziehen, bei dem die Destillation des MDACH-Ausgangsgemischs stattfindet. Die Siedepunkte der Isomere des MDACH liegen sehr dicht beieinander. Sollte bei einem bestimmten Druck das cis,cis-2,6-Diamino-1-methylcyclohexan nicht den größten Siedepunkt haben, so liegen die Differenzen der Siedepunkte des Hilfsstoffs und der im MDACH-Ausgangsgemisch enthaltenen Isomere des 2,4- und des 2,6-MDACH daher immer in der Größenordnung von mindestens 5 °C. Eine ausreichende Trennbarkeit von Hilfsstoff und MDACH ist daher gegeben.

Grundsätzlich kommen Hilfsstoffe in Frage, die mit den cis-Isomeren des MDACH ein Schwersiederazeotrop bilden.

Bevorzugt werden solche Hilfsstoffe eingesetzt, die bei einem Druck von 1 bar einen Schmelzpunkt von weniger als 60 °C, bevorzugt weniger als 10 °C, besonders bevorzugt weniger als 0 °C und ganz besonders bevorzugt weniger als - 5 °C besitzen. Ein niedriger Schmelzpunkt hat den Vorteil, dass der Hilfsstoff bei den typischen Destillationsbedingungen als Flüssigkeit vorliegt und nicht erst aufgeschmolzen werden muss. Insbesondere im Hinblick auf ein kontinuierliches Verfahren haben solche Hilfsstoffe den weiteren Vorteil, dass der Hilfsstoff in den Rohrleitungen der Anlage nicht wieder fest wird. Dies müssten ansonsten durch Beheizen der Rohrleitungen verhindert werden, was zu höheren laufenden und fixen Kosten führen würde.

Die molare Masse des Hilfsstoffs beträgt 62 bis 500 g/mol, bevorzugt 75 bis 400 g/mol, besonders bevorzugt 76 bis 300 g/mol und ganz besonders bevorzugt 100 bis 250 g/mol oder sogar 120 bis 200 g/mol.

Bevorzugt hat der Hilfsstoff neben den funktionellen Gruppen keine weiteren Heteroatome oder 1 bis 3 Ethergruppe(n) und ansonsten, neben den funktionellen Gruppen, keine weiteren Heteroatome. Unter dem Begriff Heteroatom sind solche Atome zu verstehen, die kein Kohlenstoff oder Wasserstoff sind. Bevorzugt besitzt der Hilfsstoff 1 bis 2 oder sogar eine Ethergruppe(n) und ansonsten, neben den funktionellen Gruppen, keine weiteren Heteroatome. Ebenfalls bevorzugt besitzt der Hilfsstoff neben den funktionellen Gruppen keine weiteren Heteroatome. In diesem Sinne bevorzugt sind beispielsweise Alkanole und Cycloalkanole, insbesondere Alkanole. Als Hilfsstoffe die Ethergruppen enthalten sind beispielsweise Diethylenglykol, Triethylenglykol, Dipropylenglykol oder 4-(2-Hydroxyethyl)morpholin zu nennen.

Bevorzugt besitzt der Hilfsstoff entweder

- 2 bis 4, bevorzugt 2 bis 3, besonders bevorzugt zwei Alkoholgruppen,
- eine primäre, sekundäre, oder tertiäre Aminogruppe und 1 bis 3 beziehungsweise 1 bis 2 oder genau eine Alkoholgruppe(n), oder
- 2 bis 4, bevorzugt 2 bis 3, besonders bevorzugt zwei funktionelle Gruppen, bei denen es sich jeweils unabhängig voneinander um eine primäre, sekundäre, oder tertiäre Aminogruppe handelt.

Besonders bevorzugt besitzt der Hilfsstoff entweder

- 2 bis 4, bevorzugt 2 bis 3, besonders bevorzugt zwei Alkoholgruppen, oder

- eine primäre, sekundäre, oder tertiäre Aminogruppe und 1 bis 3 beziehungsweise 1 bis 2 oder genau eine Alkoholgruppe(n).

Bevorzugt stehen im Hilfsstoff mindestens zwei der beziehungsweise, für den Fall das der Hilfsstoff genau zwei funktionelle Gruppen besitzt, beide funktionellen Gruppen in 1,2-, 1,3-, 1,4- oder 1,5-Stellung zueinander. Hierunter ist die relative Stellung der funktionellen Gruppen zueinander zu verstehen. Im Sinne der vorliegenden Erfindung ist beispielsweise sowohl 1,3-Propandiol als auch 2,4-Pentandiol ein 1,3-Diol. Besonders bevorzugt stehen im Hilfsstoff mindestens zwei der beziehungsweise, für den Fall das der Hilfsstoff genau zwei funktionelle Gruppen besitzt, beide funktionellen Gruppen in 1,2-, 1,3- oder 1,4-Stellung zueinander.
Eine gute Trennung lässt sich unter anderem dann erreichen, wenn der Hilfsstoff zwei bis vier Alkoholgruppen besitzt.
In einer in diesem Sinne bevorzugten Ausführungsform handelt es sich bei dem Hilfsstoff um eine organische Verbindung, mit

- ◦ einer molaren Masse von 62 bis 250 g/mol
- ◦ einem Siedepunkt, der mindestens 5 °C über dem Siedepunkt von cis,cis-2,6-Diamino-1-methylcyclohexan liegt, wobei sich die Siedepunkte jeweils auf einen Druck von 50 mbar beziehen,
- ◦ einem Schmelzpunkt von weniger als 60 °C bei einem Druck von 1 bar, und
- ◦ 2 bis 4 Alkohlgruppen,

wobei der Hilfsstoff neben den Alkohlgruppen keine weiteren Heteroatme oder eine oder zwei Ethergruppen und ansonsten, neben den Alkoholgruppen, keine weiteren Heteroatome besitzt.
Das oben bezüglich des Siede- und Schmelzpunkts gesagte gilt ebenso für diese Ausführungsform.
Die molare Masse dieser organischen Verbindung liegt bevorzugt bei 76 bis 200 g/mol, besonders bevorzugt 90 bis 170 g/mol und ganz besonders bevorzugt bei 90 bis 100 g/mol.
Ferner bevorzugt handelt es sich hier um eine aliphatische Verbindung, die sowohl gesättigt als auch ungesättigt sein kann und besonders bevorzugt acyclisch ist.
Ganz besonders bevorzugt handelt es sich bei einem solchen Hilfsstoff um eine Verbindung mit zwei oder drei Alkoholgruppen oder sogar um eine Verbindung mit genau zwei Alkoholgruppen, also ein Diol.
Besonders bevorzugt stehen hier mindestens zwei der beziehungsweise, für den Fall das der Hilfsstoff genau zwei Alkoholgruppen besitzt, beide Alkoholgruppen in 1,3- oder 1,4-Stellung zueinander.
Als Verbindung mit vier OH-Gruppen ist beispielsweise Diglycerin zu nennen. Bevorzugt einzusetzende Triole (Verbindungen mit drei OH-Gruppen) sind beispielsweise Glycerin, Trimethylolpropan, 1,3,6-Hexantriol, und 1,2,6-Hexantriol.
Besonders bevorzugt handelt es sich bei dem Hilfsstoff um ein Diol, insbesondere ein aliphatisches Diol. Besonders bevorzugte Diole sind Alkandiole oder Cycloalkandiole. Besonders bevorzugt sind hier die $C_3$ bis $C_{10}$-, ganz besonders bevorzugt die $C_3$ bis $C_6$-Alkandiole. Die Alkandiole sind bevorzugt unverzweigt.
Geeignete Diole sind beispielsweise 1,4-Butandiol, 1,4-Butendiol aber auch vom Ethylenglycol abgeleitete Ether wie beispielswiese Diethylenglykol oder Triethylenglykol.
Bevorzugt handelt es sich bei dem Hilfsstoff um ein 1,2-Diol insbesondere ein aliphatisches 1,2-Diol, wobei unter einem 1,2-Diol eine Verbindung verstanden wird, in der sich die OH-Gruppen in vicinaler Stellung zueinander befinden. Besonders bevorzugte 1,2-Diole sind 1,2-Alkandiole oder 1,2-Cycloalkandiole. Besonders bevorzugt sind hier die $C_4$ bis $C_{10}$-, ganz besonders bevorzugt die $C_4$ bis $C_6$-1,2-Alkandiole. Die 1,2-Alkandiole sind bevorzugt unverzweigt.
Geeignete cyclische 1,2-Diole sind beispielsweise 1,2-Cyclohexandiol und 1,2-Cyclohexandimethanol. Geeignete 1,2-Alkandiole sind beispielsweise 1,2-Hexandiol und 1,2-Pentandiol.
Eine ganz besonders gute Trennung lässt sich erreichen, wenn sich die OH-Gruppen des Hilfsstoffs in 1,3-Stellung zueinander befinden. Bevorzugt handelt es sich bei dem Hilfsstoff daher um ein 1,3-Diol insbesondere ein aliphatisches 1,3-Diol. Besonders bevorzugte 1,3-Diole sind 1,3-Alkandiole oder 1,3-Cycloalkandiole. Besonders bevorzugt sind hier die $C_3$ bis $C_{10}$-, ganz besonders bevorzugt die $C_3$ bis $C_6$-1,3-Alkandiole. Die 1,3-Alkandiole sind bevorzugt unverzweigt.
Geeignete verzweigte 1,3-Alkandiole sind beispielsweise 2-Methyl-1,3-Propandiol oder Neopentylglykol. Geeignete unverzweigte 1,3-Alkandiole sind beispielsweise 1,3-Propandiol, 1,3-Butandiol, 1,3-Pentandiol, 2,4-Pentandiol, 1,3-Hexandiol und 2,4-Hexandiol.
In besonderem Maße bevorzugt sind Diole, bei denen es sich um ein $C_3$-$C_{10}$-1,3-Alkandiol oder ein $C_4$-$C_{10}$-1,4-Alkandiol handelt.
Eine gute Trennung lässt sich auch erreichen, wenn der Hilfsstoff eine primäre, sekundäre, oder tertiäre Aminogruppe und 1 bis 3 Alkoholgruppe(n), enthält, es sich also um einen Aminoalkohol handelt. Geeignete Aminoalkohole sind beispielsweise Diethanolamin und Triethanolamin. Ein in diesem Sinne geeigneter Aminoalkohol ist beispielsweise

1,3-Aminopropanol.

In einer in diesem Sinne bevorzugten Ausführungsform handelt es sich bei dem Hilfsstoff um eine organische Verbindung, mit

- ◦ einer molaren Masse von 75 bis 300 g/mol
- ◦ einem Siedepunkt, der mindestens 5 °C über dem Siedepunkt von cis,cis-2,6-Diamino-1-methylcyclohexan liegt, wobei sich die Siedepunkte jeweils auf einen Druck von 50 mbar beziehen,
- ◦ einem Schmelzpunkt von weniger als 60 °C bei einem Druck von 1 bar, und
- ◦ einer primäre, sekundären, oder tertiären Aminogruppe und 1 bis 3 Alkoholgruppe(n),

wobei der Hilfsstoff neben den funktionellen Gruppen keine weiteren Heteroatme oder eine Ethergruppe und ansonsten, neben den funktionellen Gruppen, keine weiteren Heteroatome besitzt.

Unter einer funktionellen Gruppe im Sinne dieser Ausführungsform ist eine Alkohol-, primäre, sekundäre, oder tertiäre Aminogruppe zu verstehen.

Das oben bezüglich des Siede- und Schmelzpunkts gesagte gilt ebenso für diese Ausführungsform.

Ein solcher Hilfsstoff hat eine primäre, sekundären, oder tertiären Aminogruppe und 1 bis 3, 1 bis 2 oder eine Alkoholgruppe(n).

Die molare Masse dieser organischen Verbindung liegt bevorzugt bei 100 bis 250 g/mol, besonders bevorzugt 110 bis 200 g/mol und ganz besonders bevorzugt 115 bis 180 g/mol.

Bevorzugt handelt es sich bei einem solchen Hilfsstoff um eine aliphatische, insbesondere eine gesättigte aliphatische Verbindung. Infrage kommen hierbei sowohl cyclische als auch acyclische Verbindungen. Ebenso kann es sich aber auch um eine organische Verbindung handeln, die ein aromatisches Ringsystem, insbesondere einen Tolyl- oder Phenylrest, enthält.

Eine gute Trennung lässt sich ebenfalls erreichen, wenn der Hilfsstoff 2 bis 4 funktionelle Gruppen besitzt, bei denen es sich jeweils unabhängig voneinander um eine primäre, sekundäre, oder tertiäre Aminogruppe handelt.

In einer in diesem Sinne bevorzugten Ausführungsform handelt es sich bei dem Hilfsstoff um eine organische Verbindung, mit

- ◦ einer molaren Masse von 100 bis 300 g/mol
- ◦ einem Siedepunkt, der mindestens 5 °C über dem Siedepunkt von cis,cis-2,6-Diamino-1-methylcyclohexan liegt, wobei sich die Siedepunkte jeweils auf einen Druck von 50 mbar beziehen, und
- ◦ einem Schmelzpunkt von weniger als 60 °C bei einem Druck von 1 bar, und
- ◦ 2 bis 4 funktionellen Gruppen, bei denen es sich jeweils unabhängig voneinander um eine primäre, sekundäre, oder tertiäre Aminogruppe handelt,

wobei der Hilfsstoff neben den funktionellen Gruppen keine weiteren Heteroatme besitzt.

Unter einer funktionellen Gruppe im Sinne dieser Ausführungsform ist eine primäre, sekundäre, oder tertiäre Aminogruppe zu verstehen.

Das oben bezüglich des Siede- und Schmelzpunkts gesagte gilt ebenso für diese Ausführungsform.

Die molare Masse dieser organischen Verbindung liegt bevorzugt bei 120 bis 250 g/mol, besonders bevorzugt 150 bis 200 g/mol und ganz besonders bevorzugt 150 bis 180 g/mol.

Ein solcher Hilfsstoff hat bevorzugt 2 bis 3, besonders bevorzugt zwei funktionelle Gruppen bei denen es sich unabhängig voneinander um eine primäre, sekundäre oder tertiäre Aminogruppe handelt.

Bevorzugt handelt es sich hier bei einem solchen Hilfsstoff um eine aliphatische, insbesondere eine cycloaliphatische Verbindung die bevorzugt gesättigt ist.

Als geeignete Hilfsstoffe kommen die folgenden Verbindungen in Frage:
Ethylenglycol, 1,2-Propandiol, 2-Methylpropan-1,3-diol, 1,2-Butandiol, 2,3-Butandiol, 2-Methylbutan-1,2-diol, 3-Methylbutan-1,2-diol, 3-Methyl-1,3-butandiol, 1,2-Pentandiol, 1,3-Pentandiol, 2,4-Pentandiol, 2,3-Pentandiol, 1,2-Hexandiol, cis-1,2-Cyclopentandiol, trans-1,2-Cyclopentandiol, Cis-1,2-Cyclohexandiol, trans-1,2-Cyclohexandiol, 1,3-Propandiol, 2-Methyl-1,3-propandiol, 2,2,-Dimethyl-1,3-propandiol (Neopentylglycol), 1,3-Butandiol, 1,2-Pentandiol, 2,4-Pentandiol, 1,5-Pentandiol,1,3-Hexandiol, 2,4-Hexandiol, 1,3-Cyclobutandiol, 1,3-Cyclopentandiol, 1,3-Cyclohexandiol, cis- und trans-1,4-Butendiol, 1,4-Butandiol, 2,3-Dimethyl-1,4-Butandiol, 2,2-Dimethyl-1,4-Butandiol, 1,4-Pentandiol, 2,3-Dimethyl-1,5-pentandiol, 1,4-Hexandiol, 1,4-Cyclohexandiol, 1,3,6-Hexantriol, 1,2,3-Hexantriol, 1,2,6-Hexantriol, Glycerin, Diglycerin, Sorbitol, Pentaerythrit, Diethylenglykol, Triethylenglycol, Dipropylenglycol.

Diethanolamin, N-Methyldiethanolamin, N-Propyldiethanolamin, N-Butyldiethanolamin, Triethanolamin, N-Ethylpropanolamin, N-Propylethanolamin, N,N-Dipropylethanolamin, N-Butylethanolamin, N,N-Dibutylethanolamin, Propanolamin, Dipropanolamin, N-Methyldipropanolamin, N-Propyldipropanolamin, N-Butyldipropanolamin, Tripropanolamin, , Diisopropanolamin, N-Methyldiisopropanolamin, Triisopropanolamin, , N-2-Methylaminopropanol, 4-Amino-1-butanol, 4-(2-Hydroxyethyl)morpholin, Pentanolamin, Hydroxyethylpiperazin, N-(2-Hydroxyethyl)anilin, N,N-Di-(2-hydroxyethyl)anilin, 3-Amino-1-propanol.

2-(Diisopropylamino)ethylamine, 3-(Cyclohexylamino)proplyamin, , Dipropylentriamin, Triethylentetramin, Pentamethyldiethylentriamin, 3-(2-aminoethylamino)propylamin, Diethylentriamin, Isophorondiamin.

Bevorzugte Hilfsstoffe sind die folgenden Verbindungen:
Ethylenglycol, 1,2-Propandiol, 2-Methylpropan-1,3-diol, 1,2-Butandiol, 2,3-Butandiol, 2-Methylbutan-1,2-diol, 3-Methylbutan-1,2-diol, 3-Methyl-1,3-butandiol, 1,2-Pentandiol, 1,3-Pentandiol, 2,4-Pentandiol, 2,3-Pentandiol, 1,2-Hexandiol, cis-1,2-Cyclopentandiol, trans-1,2-Cyclopentandiol, 1,3-Propandiol, 1,5-Pentandiol, 2-Methyl-1,3-propandiol, 1,3-Butandiol, 1,2-Pentandiol, 2,4-Pentandiol, 1,3-Hexandiol, 2,4-Hexandiol, 1,3-Cyclobutandiol, 1,3-Cyclopentandiol, 1,3-Cyclohexandiol, cis- und trans-1,4-Butendiol, 1,4-Butandiol, 2,3-Dimethyl-1,4-Butandiol, 2,2-Dimethyl-1,4-Butandiol, 1,4-Pentandiol, 2,3-Dimethyl-1,5-pentandiol, 1,4-Hexandiol, 1,3,6-Hexantriol, 1,2,6-Hexantriol, Glycerin, Diglycerin, Diethylenglykol, Triethylenglycol, Dipropylenglycol.

Diethanolamin, N-Methyldiethanolamin, N-Propyldiethanolamin, N-Butyldiethanolamin, Triethanolamin, N-Ethylpropanolamin, N-Propylethanolamin, N,N-Dipropylethanolamin, N-Butylethanolamin, N,N-Dibutylethanolamin, Propanolamin, Dipropanolamin, N-Methyldipropanolamin, N-Propyldipropanolamin, N-Butyldipropanolamin, Tripropanolamin, , Diisopropanolamin, N-Methyldiisopropanolamin, Triisopropanolamin, N-2-Methylaminopropanol, 4-Amino1-butanol, 4-(2-Hydroxyethyl)morpholin, Pentanolamin, Hydroxyethylpiperazin, N-(2-Hydroxyethyl)anilin, N,N-Di-(2-hydroxyethyl)anilin, 3-Amino-1-propanol.

2-(Diisopropylamino)ethylamine, 3-(Cyclohexylamino)proplyamin, Dipropylentriamin, Triethylentetramin, Pentamethyldiethylentriamin, 3-(2-aminoethylamino)propylamin, Diethylentriamin, Isophorondiamin.

Besonders bevorzugt sind die folgenden Hilfsstoffe:
2-Methylpropan-1,3-diol, 1,2-Hexandiol, cis-1,2-Cyclopentandiol, trans-1,2-Cyclopentandiol, 1,3-Propandiol, 1,5-Pentandiol, 2-Methyl-1,3-propandiol, 1,3-Hexandiol, 2,4-Hexandiol, 1,3-Cyclobutandiol, 1,3-Cyclopentandiol, 1,3-Cyclohexandiol, cis-und trans-1,4-Butendiol, 1,4-Butandiol, 2,3-Dimethyl-1,4-Butandiol, 2,2-Dimethyl-1,4-Butandiol, 1,4-Pentandiol, 2,3-Dimethyl-1,5-pentandiol, 1,4-Hexandiol, 1,3,6-Hexantriol, 1,2,6-Hexantriol, Glycerin, Diglycerin, Diethylenglykol, Triethylenglycol, Dipropylenglycol.

Diethanolamin, N-Methyldiethanolamin, N-Propyldiethanolamin, N-Butyldiethanolamin, Triethanolamin, N-Ethylpropanolamin, N-Propylethanolamin, N,N-Dipropylethanolamin, N,N-Dibutylethanolamin, Dipropanolamin, N-Methyldipropanolamin, N-Propyldipropanolamin, N-Butyldipropanolamin, Tripropanolamin, , Diisopropanolamin, N-Methyldiisopropanolamin, Triisopropanolamin, N-2-Methylaminopropanol, 4-(2-Hydroxyethyl)morpholin, Pentanolamin, Hydroxyethylpiperazin, N-(2-Hydroxyethyl)anilin, N,N-Di-(2-hydroxyethyl)anilin.

3-(Cyclohexylamino)proplyamin, Dipropylentriamin, Triethylentetramin, 3-(2-aminoethylamino)propylamin, Isophorondiamin.

Ganz besonders bevorzugt sind Glycerin, 1,3-Propandiol, 1,4-Butandiol, cis-1,4-Butendiol, Triethylenglykol, Diglycerin, 1,5-Pentandiol, 4-(2-Hydroxyethyl)morpholin, N-(2-Hydroxyethyl)-aniln, Triethanolamin, N-Methyldiethanolamin.

Ganz besonders bevorzugt sind insbesondere 1,3-Propandiol und 1,4-Butandiol.

Das MDACH-Ausgangsgemisch enthält 0 bis 100 Gew.-% 2,4-Diamino-1-methylcyclohexan (2,4-MDACH) und 0 bis 100 Gew.-% 2,6-Diamino-1-methylcyclohexan (2,6-MDACH), bezogen auf die im MDACH-Ausgangsgemisch enthaltende Gesamtmenge an MDACH (= 2,4- und 2,6-MDACH). Des Weiteren enthält das MDACH-Ausgangsgemisch sowohl trans- als auch cis-Isomere.

Auch kann das MDACH-Ausgangsgemisch weitere Isomere des Diamino-1-methylcyclohexans enthalten, vor allem vicinale Diamino-1-methylcyclohexan-Verbindungen, wie zum Beispiel 2,3-Diaminomethylcyclohexan und 3,4-Diaminomethylcyclohexan. Der Anteil der weiteren Isomere des Diamino-1-methylcyclohexans beträgt üblicherweise 0 bis 1 Gew.-%, insbesondere weniger als 0,5 Gew.-%, bezogen auf das MDACH-Ausgangsgemisch.

Der Begriff MDACH bezeichnet alle im MDACH-Ausgangsgemisch enthaltenen Isomere des 2,4- und 2,6-MDACH, unabhängig davon, ob es sich um ein cis- oder ein trans-Isomer handelt. Entsprechendes gilt ebenso für alle Übrigen im Rahmen dieser Anmeldung offenbarten MDACH-haltigen Gemische, insbesondere cis- und trans-angereichertes MDACH.

MDACH ist die Bezugsgröße zur Angabe der gewichtsprozentualen Anteile von 2,4- und 2,6-MDACH im MDACH-Ausgangsgemisch. Folglich addieren sich die gewichtsprozentualen Angaben für 2,4- und 2,6-MDACH immer zu 100 Gew.-%. Entsprechendes gilt ebenso für alle Übrigen im Rahmen dieser Anmeldung offenbarten MDACH-haltigen Gemische, insbesondere cis-und trans-angereichertes MDACH.

Die Herstellung des MDACH-Ausgangsgemischs erfolgt üblicherweise durch Hydrierung von 2,4- oder 2,6-Toluoldiamin beziehungsweise eines Gemischs von 2,4-Toluoldiamin und 2,6-Toluoldiamin. Als Nebenprodukte treten hier unter anderem Leichtsieder auf, die durch anschließende Destillation größtenteils abgetrennt werden können. Das so erhaltene MDACH-Ausgangsgemisch kann noch geringe Mengen an Verunreinigungen, insbesondere Methylcyclohexylamine (MCHA) aber auch vicinale Diamino-1-methylcyclohexan-Verbindungen, wie beispielsweise 2,3-Diaminomethylcyclohexan und 3,4-Diaminomethylcyclohexan, enthalten. Üblicherweise enthält das MDACH-Ausgangsgemisch die folgenden Isomere.

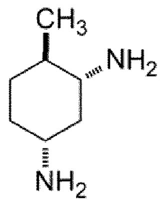

**1**

2,4-Isomer

cis

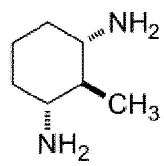

**2**

2,6-Isomer

cis

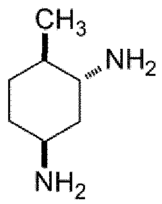

**3**

2,4-Isomer

trans

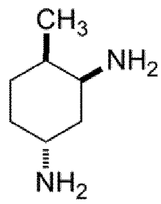

**4**

2,4-Isomer

trans

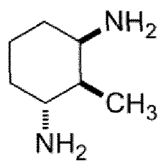

**5**

2,6-Isomer

trans

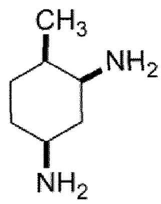

**6**

2,4-Isomer

cis

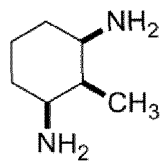

**7**

2,6-Isomer

cis

Dargestellt sind nur Diastereomere. Bei den Verbindungen 2 und 7 handelt es sich um meso-Formen. Zu allen übrigen Verbindungen existiert jeweils noch ein entsprechendes Enantiomer, so dass es insgesamt 12 verschiedene Isomere gibt.

Das MDACH-Ausgangsgemisch kann beispielsweise 0 Gew.-% 2,4-MDACH und 100 Gew.-% 2,6-MDACH, jeweils bezogen auf die im MDACH-Ausgangsgemisch enthaltende Gesamtmenge an MDACH, enthalten. Ebenso kann es 100 Gew.-% 2,4-MDACH und 0 Gew.-% 2,6-MDACH, jeweils bezogen auf die im MDACH-Ausgangsgemisch enthaltende Gesamtmenge an MDACH, enthalten. Es kann also ausschließlich 2,4-MDACH oder 2,6-MDACH enthalten. Darüber hinaus kann es auch 2,4- und 2,6-MDACH enthalten. Dies hängt im Wesentlichen davon ab, ob im Herstellungsverfahren 2,4-, 2,6- oder ein Gemisch aus 2,4- und 2,6-Toluoldiamin eingesetzt wird. Grundsätzlich entstehen bei der Herstellung des MDACH-Ausgangsgemischs sowohl trans- als auch cis-Isomere. Das heißt, 2,4- und 2,6-MDACH liegen üblicherweise in beiden Konfigurationen vor.

Enthält ein Gemisch ausschließlich 2,4- oder 2,6-MDACH, so bezieht sich die Bezeichnung MDACH zwangsläufig nur auf die entsprechenden cis- und trans-Isomere des 2,4- beziehungsweise des 2,6-MDACH. Liegen 2,4- und 2,6-MDACH vor, so bezeichnet MDACH sowohl die entsprechenden cis- und trans-Isomere des 2,4- als auch die des 2,6-MDACH. Entsprechendes gilt ebenso für alle Übrigen im Rahmen dieser Anmeldung offenbarten MDACH-haltigen Gemische, insbesondere cis- und trans-angereichertes MDACH.

Das MDACH-Ausgangsgemisch enthält bevorzugt sowohl 2,4- als auch 2,6-MDACH. Insbesondere enthält das MDACH-Ausgangsgemisch 5 bis 95 Gew.-% 2,4-MDACH und 5 bis 95 Gew.-% 2,6-MDACH, bezogen auf die im MDACH-Ausgangsgemisch enthaltende Gesamtmenge an MDACH. Besonders bevorzugt enthält das MDACH-Ausgangsgemisch 50 bis 95 Gew.-% 2,4-MDACH und 5 bis 50 Gew.-% 2,6-MDACH, bezogen auf die im MDACH-Ausgangsgemisch enthaltende Gesamtmenge an MDACH.

Daneben kann das MDACH-Ausgangsgemisch weitere Verunreinigungen wie beispielsweise MCHA enthalten. Bevorzugt besteht das MDACH-Ausgangsgemisch zu mehr als 95 Gew.-%, besonders bevorzugt zu mehr als 98 Gew.-% und ganz besonders bevorzugt zu mehr als 99 oder sogar 100 Gew.-% aus MDACH.

Wie oben beschrieben, enthält das MDACH-Ausgangsgemisch sowohl cis- als auch trans-Isomere. Die Bezeichnungen "cis" und "trans" beziehen sich, sofern nichts anders angegeben, jeweils auf die Stellung der Aminogruppen relativ zueinander.

Das MDACH-Ausgangsgemisch hat bevorzugt einen Anteil an trans-Isomeren von 5 bis 60 Gew.-% und einen

Anteil an cis-Isomeren von 40 bis 95 Gew.-%, besonders bevorzugt einen Anteil an trans-Isomeren von 10 bis 55 Gew.% und einen Anteil an cis-Isomeren von 45 bis 90 Gew.-% sowie ganz besonders bevorzugt einen Anteil an trans-Isomeren von 20 bis 50 Gew.- %und einen Anteil an cis-Isomeren von 50 bis 80 Gew.-% oder sogar einen Anteil an trans-Isomeren von 35 bis 50 Gew.-% und einen Anteil an cis-Isomeren von 50 bis 65 Gew.-%, jeweils bezogen auf die im MDACH-Ausgangsgemisch enthaltende Gesamtmenge an MDACH.

Der Anteil an cis- (cis-Anteil) und trans-Isomeren (trans-Anteil) im MDACH Ausgangsgemisch lässt sich nach der folgenden Formel berechnen:

$$cis-Anteil=\frac{[cis]}{[trans]+[cis]}\cdot 100\,Gew.-\%$$

$$trans-Anteil=\frac{[trans]}{[trans]+[cis]}\cdot 100\,Gew.-\%$$

Dabei gilt cis-Anteil + trans-Anteil = 100 Gew.-%.

Der Ausdruck [cis] bezeichnet die Gesamtmasse (=Gesamtmenge) der im MDACH-Ausgangsgemisch enthaltenen cis-Isomere, unabhängig davon, ob es sich um 2,4- oder um 2,6-MDACH handelt. Dementsprechend bezeichnet der Ausdruck [trans] die Gesamtmasse der im MDACH-Ausgangsgemisch enthaltenen trans-Isomere, unabhängig davon, ob es sich um 2,4- oder um 2,6-MDACH handelt. Folglich bezeichnet die Summe [trans] + [cis] die Gesamtmasse des im MDACH-Ausgangsgemisch enthaltenen MDACH. MDACH dient als Bezugsgröße zur Angabe der gewichtsprozentualen Anteile der cis- und trans-Isomere im MDACH-Ausgangsgemisch. Folglich addieren sich die gewichtsprozentualen Angaben für die cis- und trans-Isomere des MDACH immer zu 100 Gew.-%.

Die obigen Formeln zur Berechnung des cis- und trans-Anteils gelten ebenso für alle übrigen im Rahmen dieser Anmeldung offenbarten MDACH-haltigen Gemische, insbesondere cis- und trans-angereichertes MDACH.

Bei der erfindungsgemäßen Destillation des MDACH-Ausgangsgemischs wird trans-angereichertes MDACH abdestilliert. Hierdurch reichert sich das cis-Isomer im Sumpf an. Des Weiteren enthält das Sumpfprodukt üblicherweise den überwiegenden Teil des Hilfsstoffs.

Das erfindungsgemäße Verfahren kann sowohl kontinuierlich als auch diskontinuierlich durchgeführt werden.

Die erfindungsgemäße Destillation des MDACH-Ausgangsgemischs in Gegenwart eines Hilfsstoffes erfolgt üblicherweise mithilfe von Verdampfern und/oder Kolonnen. Hierfür eignen sich die dem Fachmann bekannten Kolonnen.

Bevorzugt sind Packungskolonnen mit strukturierten Packungen oder Füllkörpern, Bodenkolonnen mit Böden wie Siebböden, Glockenböden oder Ventilböden. Als Verdampfer kommen alle üblichen Varianten wie beispielsweise Umlauf-, Dünnschicht- und Fallfilmverdampfer in Frage. Die Kolonnen können als konventionelle Kolonnen, als Kolonnen mit Seitenabzug oder als Trennwandkolonnen konzipiert werden.

Bei kontinuierlicher Fahrweise der Destillation wird die Kolonne bevorzugt so eingestellt, dass ein Rücklaufverhältnis (Rücklauf/Destillatabzug) im Bereich von 0,1 bis unendlich vorzugsweise von 1 bis 10 resultiert.

Geeignete Kolonnen haben bevorzugt eine theoretische Stufenzahl ($N_{th}$) von 10 bis 150, besonders bevorzugt von 30 bis 110.

Das erfindungsgemäße Destillationsverfahren wird vorzugsweise bei einer Kopftemperatur von 30 bis 280 °C und einem Kopfdruck von 1 bis 1000 mbar, besonders bevorzugt bei einer Kopftemperatur von 70 bis 220 °C und einem Kopfdruck von 5 bis 800 mbar und ganz besonders bevorzugt bei einer Kopftemperatur von 80 bis 180 °C und einem Kopfdruck von 10 bis 500 mbar oder sogar bei einer Kopftemperatur von 80 bis 144 °C und einem Kopfdruck von 10 bis 150 mbar durchgeführt.

Vorzugsweise wird die erfindungsgemäße Destillation in Abwesenheit von Sauerstoff durchgeführt. Unter Abwesenheit wird hier verstanden, dass der Sauerstoffvolumenanteil kleiner als 0,1%, insbesondere kleiner 0,01 und ganz besonders kleiner 0,001 %, bezogen auf das Gesamtvolumen der Destillationskolonne ist.

Das Stoffmengenverhältnis von Hilfsstoffs und MDACH, ist bevorzugt größer als 0,2, besonders bevorzugt 0,3 bis 5 und ganz besonders bevorzugt 0,5 bis 2. Im Fall der diskontinuierlichen Fahrweise werden das MDACH-Ausgangsgemisch und der Hilfsstoff im entsprechenden Verhältnis in der Destillationsblase vorgelegt. Bei der kontinuierlichen Reaktionsführung wird das entsprechende Verhältnis über die Zulaufströme des MDACH-Ausgangsgemischs und des Hilfsstoffs eingestellt.

Definition 'Stoffmengenverhältnis': (Stoffmenge des Hilfsstoffs) / (Stoffmenge der Gesamtmenge des im MDACH-Ausgangsgemisch enthaltenen MDACH). Unter Stoffmenge ist die Molmenge zu verstehen.

Bei der diskontinuierlichen Führung können das MDACH-Ausgangsgemisch und der Hilfsstoff in einer Destillationsblase, gegebenenfalls mit aufgesetzter Kolonne, vorzugsweise unter Luftausschluss, vorgelegt und gemeinsam aufgeheizt werden. Während der Destillation können einzelne Fraktion des trans-angereicherten MDACH entnommen

werden.

Bevorzugt wird die Destillation des MDACH-Ausgangsgemischs in Gegenwart eines Hilfsstoffs kontinuierlich durchgeführt.

Besonders bevorzugt wird das erfindungsgemäße Verfahren kontinuierlich durchgeführt und umfasst die folgenden Schritte:

a) Zuführen des Hilfsstoffs in eine Kolonne (K),
b) Zuführen des MDACH-Ausgangsgemischs in K,
c) Abdestillieren von trans-angereichertem MDACH aus K.

Als Kolonne (K) werden bevorzugt die oben genannten Kolonnentypen eingesetzt. K hat bevorzugt eine theoretische Stufenzahl ($N_{th}$) von 10 bis 150, besonders bevorzugt von 30 bis 110.

Üblicherweise ist die Kolonne (K) mit einem Kondensator und einem Verdampfer ausgestattet.

In den Schritten a) und b) werden das MDACH-Ausgangsgemisch und der Hilfsstoff bevorzugt im Verstärker- oder Abtriebsteil in K zugeführt. Dabei können sie gemeinsam in K zugeführt werden. Bevorzugt werden das MDACH-Ausgangsgemisch und der Hilfsstoff dabei zunächst vermischt und anschließend in K zugeführt.

Besonders bevorzugt werden das MDACH-Ausgangsgemisch und der Hilfsstoff getrennt voneinander in K zugeführt. Ganz besonders bevorzugt erfolgt die Zuführung des Hilfsstoffs oberhalb der Zuführung des MDACH-Ausgangsgemischs. Insbesondere erfolgt die Zuführung des Hilfsstoffs dabei im oberen Viertel der theoretischen Stufen der Kolonne und die Zuführung des MDACH-Ausgangsgemischs im unteren Viertel der theoretischen Stufen der Kolonne. Ganz besonders bevorzugt erfolgt die Zuführung des Hilfsstoffs mindestens 10 oder sogar mindestens 20 theoretische Stufen oberhalb der Zuführung des MDACH-Ausgangsgemischs.

Die Zuführung des Hilfsstoffs und des MDACH-Ausgangsgemischs in den Schritten a) und b) kann sowohl flüssig, gasförmig, als auch flüssig siedend erfolgen.

Besitzt eine Kolonne beispielsweise 40 theoretische Stufen, so stellen die ersten 10 theoretischen Stufen, ausgehend vom unteren Ende der Kolonne, das erste Viertel, die Stufen 11 bis 20 das zweite Viertel, die Stufen 21 bis 30 das dritte Viertel und die Stufen 31 bis 40 das letzte beziehungsweise obere Viertel der Kolonne dar. Entsprechendes gilt für andere Aufteilungen einer Kolonne, beispielsweise Drittel (siehe unten).

In Schritt c) wird trans-angereichertes MDACH üblicherweise am Kopf der Kolonne als Destillat oder als Seitenstrom abgezogen. Wird das trans-angereicherte MDACH im Seitenstrom abgezogen, so befindet sich die Seitenabzugsstelle zur Entfernung des trans-angereicherten MDACH aus K gemäß Schritt c) bevorzugt mindestens 1, besonders bevorzugt mindestens 5 theoretische Stufen über der Zulaufstelle des Hilfsstoffs.

Eine bevorzugte Ausführungsform des erfindungsgemäßen Verfahrens ist in Abbildung 1 dargestellt. Die Kolonne (K) ist mit einem Verdampfer und einem Kondensator versehen. Das MDACH-Ausgangsgemisch wird im unteren und der Hilfsstoff im oberen Drittel der theoretischen Stufen der Kolonne kontinuierlich zugeführt (Schritt a) und b)). Als Destillat wird trans-angereichertes MDACH abgezogen (Schritt c)).

Das trans-angereicherte MDACH kann Verunreinigungen, insbesondere Hilfsstoff und MCHA, enthalten.

Zur Entfernung von Niedrigsiedern, also solchen Verbindungen, die einen niedrigeren Siedepunkt als MDACH aufweisen, insbesondere MCHA, ist es vorteilhaft, das trans-angereicherte MDACH im Seitenstrom abzuziehen, während am Kopfkondensator Destillat abgezogen wird. Dieses enthält neben MDACH ebenfalls Niedrigsieder (insbesondere MCHA), wodurch deren Anteil im trans-angereicherten MDACH, das im Seitenstrom abgezogen wird, verringert wird.

Weiterhin vorteilhaft ist es, wenn sich an den Kopfkondensator ein Nachkondensator anschließt. Hierbei lässt sich am Nachkondensator sogenanntes Nachkondensator-Destillat abziehen. Dieses enthält neben MDACH Niedrigsieder (insbesondere MCHA), wodurch deren Anteil im trans-angereicherten MDACH, das im Seitenstrom abgezogen wird, verringert wird.

Insbesondere kann sowohl am Kopf- als auch am Nachkondensator Destillat abgezogen werden. Dies führt zu einer weiteren Verringerung von Niedrigsiedern im trans-angereicherten MDACH, welches im Seitenstrom abgezogen wird.

Der Anteil an Hilfsstoff im trans-angereicherten MDACH ist üblicherweise dann gering, wenn eine Kolonne mit einer hohen Trennleistung eingesetzt wird und ausreichend viele Trennstufen zwischen der Zuführung des Hilfsstoffs und dem Abzug des trans-angereicherten MDACH (im Destillat und/oder Seitenabzug) liegen. Bevorzugt liegen zwischen der Zuführung des Hilfsstoffs und dem Abzug des trans-angereichertem MDACH mindestens eine theoretische Stufe, besonders bevorzugt mindestens 5 theoretische Stufen.

Eine weitere Möglichkeit zur Verringerung des Anteils an Hilfsstoff und MCHA im trans-angereicherten MDACH besteht darin, einen zweiten Destillationsschritt durchzuführen.

Bevorzugt umfasst das erfindungsgemäße kontinuierliche Verfahren zusätzlich zu den Schritten

a) bis c) die weiteren Schritte:

d) Zuführen des trans-angereicherten MDACH aus Schritt c) in eine Kolonne (K')
e) Entfernen von trans-angereichertem MDACH aus K' im Seitenstrom oder Destillat.

Hierbei weist das in Schritt e) erhaltene trans-angereicherte MDACH weniger Verunreinigungen auf, als das in Schritt c) abdestillierte trans-angereicherte MDACH.

Die Schritte d) bis e) können insbesondere dann durchgeführt werden, wenn das erhaltene trans-MDACH weiter gereinigt werden soll. Dies ist beispielsweise dann sinnvoll, wenn das in Schritt c) erhaltene trans-angereicherte MDACH mehr als 0,1 Gew.-% Hilfsstoff oder mehr als 0,1 Gew.-% MCHA, bezogen auf die Gesamtmasse des trans-angereicherten MDACHs, enthält.

Als Kolonne (K') werden bevorzugt die oben genannten Kolonnentypen eingesetzt. K' hat bevorzugt eine theoretische Stufezahl ($N_{th}$) von 10 bis 150, besonders bevorzugt von 30 bis 110.

Üblicherweise ist die Kolonne (K') mit einem Kondensator und einem Verdampfer ausgestattet.

Bevorzugt wird in Schritt d) das trans-angereicherte MDACH im mittleren Drittel der theoretischen Stufen von K' zugeführt. Ganz besonders bevorzugt ist, im Falle des Seitenabzuges des trans-angereicherten MDACHS die Seitenabzugsstelle zur Entfernung des trans-angereicherten MDACH aus K' gemäß Schritt e) mindestens 1, insbesondere mindestens 5 theoretische Stufen über der Zulaufstelle des trans-angereicherten MDACH aus Schritt c).

In Schritt e) wird trans-angereichertes MDACH entweder als Seitenstrom oder als Destillat abgezogen. Besonders bevorzugt ist der Abzug von trans-angereichertem MDACH als Destillat, wenn die MCHA-Konzentration bereits in dem Schritt c) ausreichend reduziert wurde oder MCHA gar nicht im MDACH-Ausgangsgemisch vorliegt.

Zur Entfernung von Niedrigsiedern, insbesondere MCHA, ist es vorteilhaft, am Kopf-Kondensator Destillat abzuziehen. Dieses enthält neben MDACH ebenfalls Niedrigsieder (insbesondere MCHA), wodurch deren Anteil im trans-angereicherten MDACH verringert wird.

Weiterhin vorteilhaft ist es, wenn sich an den Kopfkondensator ein Nachkondensator anschließt. Hierbei lässt sich am Nachkondensator sogenanntes Nachkondensator-Destillat abziehen. Dieses enthält neben MDACH Niedrigsieder (insbesondere MCHA), wodurch deren Anteil im trans-angereicherten MDACH verringert wird.

Ganz besonders bevorzugt wird sowohl am Kopf- als auch am Nachkondensator Destillat abgezogen werden. Dies führt zu einer weiteren Verringerung von Niedrigsiedern im trans-angereicherten MDACH.

Eine weitere besonders bevorzugte Ausführungsform des erfindungsgemäßen Verfahrens ist in Abbildung 2 dargestellt. Die Kolonnen (K und K') sind mit einem Verdampfer und einem Kopfkondensator versehen. An den Kopfkondensator von K' schließt sich ein Nachkondensator an. Das MDACH-Ausgangsgemisch wird im unteren und der Hilfsstoff im oberen Drittel der theoretischen Stufen der ersten Kolonne (K) kontinuierlich zugeführt (Schritt a) und b)). Als Destillat wird trans-angereichertes MDACH abgezogen (Schritt c)). Dieses wird einer zweiten Kolonne

(K') zugeführt (Schritt d)). Im Nachkondensator wird Nachkondensator-Destillat abgezogen. Optional kann am Kopfkondensator ebenfalls Destillat abgezogen werden. Trans-angereichertes MDACH wird im Seitenstrom abgezogen (Schritt e)).

In einer vorteilhaften Ausgestaltung des erfindungsgemäßen Verfahrens kann in einem sich anschließenden Destillationsschritt cis-angereichertes MDACH erhalten werden.

In diesem Sinne bevorzugt ist also ein Verfahren zur Herstellung von cis- und trans-angereichertem MDACH, das die folgenden Schritte umfasst:

I. Erfindungsgemäße Destillation eines MDACH-Ausgangsgemischs,
II. Destillation des in Schritt I erhaltenen Sumpfprodukts, wobei cis-angereichertes MDACH abdestilliert wird,

wobei
cis-angereichertes MDACH ein Gemisch ist, das 0 bis 100 Gew.-% 2,4-MDACH und 0 bis 100 Gew.-% 2,6-MDACH, bezogen auf die im Gemisch enthaltende Gesamtmenge an MDACH, enthält, wobei der Anteil an cis-Isomeren im Gemisch, bezogen auf die im Gemisch enthaltene Gesamtmenge an MDACH, höher ist, als der Anteil an cis-Isomeren im MDACH-Ausgangsgemisch, bezogen auf die MDACH-Ausgangsgemisch enthaltene Menge an MDACH.

Schritt I entspricht der erfindungsgemäßen Destillation eines MDACH-Ausgangsgemischs, die in Gegenwart eines Hilfsstoffs durchgeführt wird, wobei trans-angereichertes MDACH abdestilliert wird.

Die Schritte I und II erfolgen üblicherweise mithilfe von Verdampfern und/oder Kolonnen. Hierfür eignen sich die dem Fachmann bekannten Kolonnen. Bevorzugt werden die oben genannten Kolonnentypen eingesetzt. Geeignete Kolonnen haben bevorzugt eine theoretische Stufenzahl ($N_{th}$) von 10 bis 150, besonders bevorzugt von 30 bis 110.

Bei kontinuierlicher Führung werden die Kolonnen bevorzugt so eingestellt, dass ein Rücklaufverhältnis (Rücklauf/Destillatabzug) im Bereich von 0,1 bis unendlich vorzugsweise von 1 bis 10 resultiert.

Die Destillation gemäß Schritt I erfolgt wie oben beschrieben. Das heißt, sie wird vorzugsweise bei einer Kopftemperatur von 30 bis 280 °C und einem Kopfdruck von 1 bis 1000 mbar, besonders bevorzugt bei einer Kopftemperatur von 70 bis 220 °C und einem Kopfdruck von 5 bis 800 mbar und ganz besonders bevorzugt bei einer Kopftemperatur von 80 bis 180 °C und einem Kopfdruck von 10 bis 500 mbar oder sogar bei einer Kopftemperatur von 80 bis 144 °C und einem Kopfdruck von 10 bis 150 mbar durchgeführt.

Die Destillation gemäß Schritt II erfolgt unter denselben Bedingungen (Kopfdruck und Kopftemperatur) wie oben für Schritt I angegeben.

Vorzugsweise werden die Schritte I und II in Abwesenheit von Sauerstoff durchgeführt. Unter Abwesenheit wird hier verstanden, dass der Sauerstoffvolumenanteil kleiner als 0,1 %, insbesondere kleiner 0,01 und ganz besonders kleiner 0,001 %, bezogen auf das Gesamtvolumen der Destillationskolonne ist.

Das Stoffmengenverhältnis von Hilfsstoffs und MDACH, ist bevorzugt größer als 0,2, besonders bevorzugt 0,3 bis 5 und ganz besonders bevorzugt 0,5 bis 2. Im Fall der diskontinuierlichen Fahrweise werden das MDACH-Ausgangsgemisch und der Hilfsstoff im entsprechenden Verhältnis in der Destillationsblase vorgelegt. Bei der kontinuierlichen Reaktionsführung wird das entsprechende Verhältnis über die Zulaufströme des MDACH-Ausgangsgemischs und des Hilfsstoffs eingestellt.

Bei der diskontinuierlichen Führung des Schritts I können das MDACH-Ausgangsgemisch und der Hilfsstoff in einer Destillationsblase, gegebenenfalls mit aufgesetzter Kolonne, vorzugsweise unter Luftausschluss, vorgelegt und gemeinsam aufgeheizt werden. Während der Destillation können einzelne Fraktion des trans-angereicherten MDACH entnommen werden.

In einem weiteren Schritt (Schritt II), kann das in Schritt I erhaltene Sumpfprodukt weiter aufgearbeitet werden. Hierzu kann dieselbe Apparatur wie für Schritt I verwendet, das heißt Schritt II erfolgt dann durch Aufheizen des in Schritt I erhaltenen Sumpfprodukts in derselben Apparatur. Grundsätzlich kann das Sumpfprodukt aber auch in einer anderen Apparatur destilliert werden. In beiden Fällen liegt das Sumpfprodukt in einer Destillationsblase, gegebenenfalls mit aufgesetzter Kolonne, vorzugsweise unter Luftausschluss vor. Hierbei wird cis-angereichertes MDACH abdestilliert.

Weiterhin bevorzugt ist ein Verfahren zur Herstellung von cis- und trans-angereichertem MDACH, das kontinuierlich wie folgt durchgeführt wird:

In Schritt I:

a) Zuführen des Hilfsstoffs in eine Kolonne (K),
b) Zuführen des MDACH-Ausgangsgemischs in K,
c) Abdestillieren von trans-angereichertem MDACH aus K.

In Schritt II:

a) Zuführen des Sumpfprodukts aus K in eine zweite Kolonne (K2),
b) Abdestillieren von cis-angereichertem MDACH aus K2.

Als Kolonnen K und K2 werden bevorzugt die oben genannten Kolonnentypen eingesetzt. K und K2 haben bevorzugt eine theoretische Stufenzahl ($N_{th}$) von 10 bis 150, besonders bevorzugt von 30 bis 110.

Üblicherweise sind die Kolonnen K und K2 mit einem Kondensator und einem Verdampfer ausgestattet.

Die Destillation in Schritt I entspricht der oben beschriebenen kontinuierlichen Destillation des MDACH-Ausgangsgemischs gemäß den Schritten a) bis c). Das heißt, die Schritte a), b) und c) entsprechen den Schritten I a), I b) und I c). Folglich beziehen sich die entsprechenden Erklärungen sowie die dort als bevorzugt, besonders bevorzugt und ganz besonders bevorzugt genannten Merkmale ebenfalls auf Schritt I. Insbesondere lässt sich das in Schritt I c) erhaltene trans-angereicherte MDACH gemäß den Schritten d) und e), dann als Schritte I d) und I e) zu bezeichnen, weiter aufarbeiten.

In Schritt II a) wird das Sumpfprodukt bevorzugt im Verstärker- oder Abtriebsteil und besonders bevorzugt im unteren Drittel der theoretischen Stufen von K2 zugeführt.

Die Zuführung des Sumpfprodukts in Schritt II a) kann sowohl flüssig, gasförmig, als auch flüssig siedend erfolgen.

In Schritt II b) wird cis-angereichertes MDACH üblicherweise am Kopf der Kolonne als Destillat oder als Seitenstrom abgezogen.

Bevorzugt wird in einem Schritt III das Sumpfprodukt aus K2 in K rückgeführt. Besonders bevorzugt wird das Sumpfprodukt in K an derselben Stelle wie der Hilfsstoff (Schritt I a)) zugeführt.

Das Sumpfprodukt aus K2 enthält bevorzugt 60 bis 100 Gew.-% Hilfsstoff und 0 bis 40 Gew.-% MDACH, besonders bevorzugt 70 bis 100 Gew.-% Hilfsstoff und 0 bis 30 Gew.-% MDACH, bezogen auf die Gesamtmasse des Sumpfprodukts. Weiterhin bevorzugt besteht das im Sumpfprodukt enthaltene MDACH zu mindestens 50, besonders bevorzugt 60 und ganz besonders bevorzugt 70 oder sogar >90 Gew.-% aus cis-Isomeren.

Durch die Rückführung des Sumpfprodukts aus K2 in K wird der Hilfsstoff im Kreis geführt. Es ist dann grundsätzlich nicht mehr notwendig, dem System konstant frischen Hilfsstoff zuzuführen. In diesem Fall wird der Hilfsstoff

dem System zunächst in der gewünschten Menge via Schritt I a) zugeführt. Sobald sich in den Kolonnen K und K2 ein Gleichgewicht eingestellt hat, kann die Zuführung an frischem Hilfsstoff entweder ganz eingestellt oder zumindest deutlich reduziert werden.

Um die Anreicherung von hochsiedenden Verunreinigungen im rückgeführten Sumpfprodukt zu verhindern, wird bevorzugt mindestens 1% des Sumpfproduktes ausgeschleust. In diesem Fall wird die Menge an frisch zugeführtem Hilfsstoff so eingestellt, dass die durch das Ausschleusen entstandenen Verluste ausgeglichen werden.

Eine besonders bevorzugte Ausführungsform des erfindungsgemäßen Verfahrens ist in Abbildung 3 dargestellt.

Der Hilfsstoff wird im oberen und das MDACH-Ausgangsgemisch im unteren Drittel der theoretischen Stufen von K zugeführt (Schritte I a) und I b)). Trans-angereichertes MDACH wird als Destillat abgezogen (Schritt I c)). Das Sumpfprodukt aus K wird einer zweiten Kolonne (K2) zugeführt (Schritt II a)). Als Destillat wird cis-angereichertes MDACH aus K2 abgezogen (Schritt II b)). Das Sumpfprodukt aus K2 wird in K rückgeführt (Schritt III). Ein Teil des Sumpfprodukts aus K2 wird ausgeschleust.

Mithilfe des erfindungsgemäßen Destillationsverfahrens lässt sich trans-angereichertes MDACH herstellen.

Trans-angereichertes MDACH ist ein Gemisch, das 0 bis 100 Gew.-% 2,4-MDACH und 0 bis 100 Gew.-% 2,6-MDACH, bezogen auf die im Gemisch enthaltende Gesamtmenge an MDACH, enthält, wobei der Anteil an trans-Isomeren im Gemisch, bezogen auf die im Gemisch enthaltene Gesamtmenge an MDACH, höher ist, als der Anteil an trans-Isomeren im MDACH-Ausgangsgemisch, bezogen auf die MDACH-Ausgangsgemisch enthaltene Menge an MDACH.

Auch kann das trans-angereicherte MDACH weitere Isomere des Diamino-1-methylcyclohexans enthalten, vor allem vicinale Diamino-1-methylcyclohexan-Verbindungen, wie zum Beispiel 2,3-Diaminomethylcyclohexan und 3,4-Diaminomethylcyclohexan. Der Anteil der weiteren Isomere des Diamino-1-methylcyclohexan beträgt üblicherweise 0 bis 1 Gew.-%, insbesondere weniger als 0,5 Gew.-%, bezogen auf das trans-angereicherte MDACH.

Die obigen Formeln zur Berechnung des cis- und des trans-Anteils gelten ebenso für das trans-angereicherte MDACH. Unter eine Erhöhung des trans-Anteils ist also zu verstehen, dass der trans-Anteil (berechnet nach obiger Formel) für das trans-angereicherte MDACH höher ist, als der trans-Anteil (berechnet nach obiger Formel) für das MDACH-Ausgangsgemisch.

Hat beispielsweise das MDACH-Ausgangsgemisch einen Anteil von 35 Gew.-% trans-Isomeren (und einen Anteil von 65 Gew.-% cis-Isomeren), so ist der Anteil an trans-Isomeren im trans-angereicherten MDACH größer als 35 Gew.-%. Zwangsläufig ist der Anteil an cis-Isomeren im trans-angereicherten MDACH geringer als 65 Gew.-%.

Das trans-angereicherte MDACH hat bevorzugt einen Anteil an trans-Isomeren von 55 bis 100 Gew.-%, besonders bevorzugt von 62 bis 85 Gew.-% und ganz besonders bevorzugt von 65 bis 80 Gew.-%, jeweils bezogen auf die im Gemisch enthaltende Gesamtmenge an MDACH.

Das trans-angereicherte MDACH kann Verunreinigungen, insbesondere MCHA und Hilfsstoff enthalten. Bevorzugt besteht das trans-angereicherte MDACH zu mehr als 95, besonders bevorzugt mehr als 98 und ganz besonders bevorzugt 99 oder sogar 100 Gew.-% aus MDACH.

Darüber hinaus enthält das trans-angereicherte MDACH bevorzugt 5 bis 95 Gew.-% 2,4-MDACH und 5 bis 95 Gew.-% 2,6-MDACH sowie besonders bevorzugt 50 bis 95 Gew.-% 2,4-MDACH und 5 bis 50 Gew.-% 2,6-MDACH, jeweils bezogen auf die im Gemisch enthaltende Gesamtmenge an MDACH.

Daneben enthält das trans-angereicherte MDACH bevorzugt höchstens 1, besonders bevorzugt weniger als 0,8 oder sogar weniger als 0,7 Gew.-% MCHA, bezogen auf die Gesamtmenge des trans-angereicherten MDACH.

Ganz besonders bevorzugt ist das trans-angereicherte MDACH frei von MCHA. Hierunter ist zu verstehen, dass der Anteil an MCHA im trans-angereicherten MDACH weniger als 0,01 Gew.-%, bezogen auf die Gesamtmenge des trans-angereicherten MDACH, beträgt.

Das trans-angereicherte MDACH enthält bevorzugt höchstens 1 und besonders bevorzugt weniger als 0,1 Gew.-% Hilfsstoff, bezogen auf die Gesamtmenge des trans-angereicherten MDACH.

Ganz besonders bevorzugt ist das trans-angereicherte MDACH frei von Hilfsstoff. Hierunter ist zu verstehen, dass der Anteil an Hilfsstoff im trans-angereicherten MDACH weniger als 0,01 Gew.-%, bezogen auf die Gesamtmenge des trans-angereicherten MDACH, beträgt.

In einer vorteilhaften Ausgestaltung des erfindungsgemäßen Verfahrens lässt sich sowohl cisals auch trans-angereichertes MDACH herstellen.

Cis-angereichertes MDACH ist ein Gemisch, das 0 bis 100 Gew.-% 2,4-MDACH und 0 bis 100 Gew.-% 2,6-MDACH, bezogen auf die im Gemisch enthaltende Gesamtmenge an MDACH, enthält, wobei der Anteil an cis-Isomeren im Gemisch, bezogen auf die im Gemisch enthaltene Gesamtmenge an MDACH, höher ist, als der Anteil an cis-Isomeren im MDACH-Ausgangsgemisch, bezogen auf die MDACH-Ausgangsgemisch enthaltene Menge an MDACH.

Auch kann das cis-angereicherte MDACH weitere Isomere des Diamino-1-methylcyclohexans enthalten, vor allem vicinale Diamino-1-methylcyclohexan-Verbindungen, wie zum Beispiel 2,3-Diaminomethylcyclohexan und 3,4-Diaminomethylcyclohexan. Der Anteil der weiteren Isomere des Diamino-1-methylcyclohexan beträgt üblicherweise 0 bis 1 Gew.-%, insbesondere weniger als 0,5 Gew.-%, bezogen auf das cis-angereicherte MDACH.

Die obige Formeln zur Berechnung des cis- und des trans-Anteils im MDACH-Ausgangsgemisch gelten ebenso für das cis-angereicherte MDACH. Unter eine Erhöhung des cis-Anteils ist also zu verstehen, dass der cis-Anteil (berechnet nach obiger Formel) für das cis-angereicherte MDACH höher ist, als der cis-Anteil (berechnet nach obiger Formel) für das MDACH-Ausgangsgemisch.
Hat beispielsweise das MDACH-Ausgangsgemisch einen Anteil von 65 Gew.-% cis-Isomeren (und einen Anteil von 35 Gew.-% trans-Isomeren), so ist der Anteil an cis-Isomeren im cis-angereicherten MDACH größer als 65 Gew.-%. Zwangsläufig ist der Anteil an trans-Isomeren im cis-angereicherten MDACH geringer als 35 Gew.-%.
Das cis-angereicherte MDACH hat bevorzugt einen Anteil an cis-Isomeren von 55 bis 100 Gew.-%, bezogen auf die im Gemisch enthaltende Gesamtmenge an MDACH. Ebenfalls bevorzugt sind ein Anteil an cis-Isomeren von 60 bis 99 Gew.-% oder 70 bis 95 Gew.-%, jeweils bezogen auf die im Gemisch enthaltende Gesamtmenge an MDACH.
Das cis-angereicherte MDACH kann Verunreinigungen, insbesondere Hilfsstoff enthalten. Bevorzugt besteht das cis-angereicherte MDACH zu mehr als 95, besonders bevorzugt mehr als 98 und ganz besonders bevorzugt 99 oder sogar 100 Gew.-% aus MDACH.
Darüber hinaus enthält das cis-angereicherte MDACH bevorzugt 5 bis 95 Gew.-% 2,4-MDACH und 5 bis 95 Gew.-% 2,6-MDACH sowie besonders bevorzugt 50 bis 100 Gew.-% 2,4-MDACH und 0 bis 50 Gew.-% 2,6-MDACH, jeweils bezogen auf die im Gemisch enthaltende Gesamtmenge an MDACH.
Das cis-angereicherte MDACH enthält bevorzugt höchstens 10 und besonders bevorzugt weniger als 5 Gew.-% Hilfsstoff, bezogen auf die Gesamtmenge des cis-angereicherten MDACH. Ganz besonders bevorzugt ist das cis-angereicherte MDACH frei von Hilfsstoff. Hierunter ist zu verstehen, dass der Anteil an Hilfsstoff im cis-angereicherten MDACH weniger als 0,01 Gew.-%, bezogen auf die Gesamtmenge des cis-angereicherten MDACH, beträgt.

Die vorliegende Offenbarung betrifft ferner trans-angereichertes MDACH, das nach dem erfindungsgemäßen Verfahren herstellbar ist. Die oben für das erfindungsgemäße Verfahren als bevorzugt, besonders bevorzugt und ganz besonders bevorzugt herausgestellten Merkmale beziehen sich ebenfalls auf das nach diesem Verfahren herstellbare trans-angereichertes MDACH.

Insbesondere betrifft die vorliegende Offenbarung trans-angereichertes MDACH, bei dem es sich um ein Gemisch handelt, das 0 bis 100 Gew.-% 2,4-MDACH und 0 bis 100 Gew.-% 2,6-MDACH, bezogen auf die im Gemisch enthaltende Gesamtmenge an MDACH, enthält und einen Anteil an trans-Isomeren von 55 bis 100 Gew.-%, bezogen auf die im Gemisch enthaltende Gesamtmenge an MDACH, hat.

Auch kann das trans-angereicherte MDACH weitere Isomere des Diamino-1-methylcyclohexans enthalten, vor allem vicinale Diamino-1-methylcyclohexan-Verbindungen, wie zum Beispiel 2,3-Diaminomethylcyclohexan und 3,4-Diaminomethylcyclohexan. Der Anteil der weiteren Isomere des Diamino-1-methylcyclohexan beträgt üblicherweise 0 bis 1 Gew.-%, insbesondere weniger als 0,5 Gew.-%, bezogen auf das trans-angereicherte MDACH.
Besonders bevorzugt hat das trans-angereicherte MDACH einen Anteil an trans-Isomeren von 62 bis 85 Gew.-% und ganz besonders bevorzugt 65 bis 80 Gew.-%, jeweils bezogen auf die im Gemisch enthaltende Gesamtmenge an MDACH.
Das trans-angereicherte MDACH besteht bevorzugt zu mehr als 95, besonders bevorzugt mehr als 98 und ganz besonders bevorzugt 99 oder sogar 100 Gew.-% aus MDACH.
Darüber hinaus enthält das trans-angereicherte MDACH bevorzugt 5 bis 95 Gew.-% 2,4-MDACH und 5 bis 95 Gew.-% 2,6-MDACH sowie besonders bevorzugt 50 bis 95 Gew.-% 2,4-MDACH und 5 bis 50 Gew.-% 2,6-MDACH, bezogen auf die im Gemisch enthaltende Gesamtmenge an MDACH.
Das trans-angereicherte MDACH enthält bevorzugt höchstens 1, besonders bevorzugt weniger als 0,8 und ganz besonders bevorzugt weniger als 0,7 Gew.-% MCHA, bezogen auf die Gesamtmenge des trans-angereichertem MDACH.
Das trans-angereicherte MDACH enthält bevorzugt höchstens 1 und besonders bevorzugt weniger als 0,1 Gew.-% Hilfsstoff, bezogen auf die Gesamtmenge des trans-angereicherten MDACH.
Ganz besonders bevorzugt ist das trans-angereicherte MDACH frei von Hilfsstoff. Hierunter ist zu verstehen, dass der Anteil an Hilfsstoff im trans-angereicherten MDACH weniger als 0,01 Gew.-%, bezogen auf die Gesamtmenge des trans-angereicherten MDACH, enthält.

Des Weiteren betrifft die vorliegende Offenbarung cis-angereichertes MDACH, das nach dem erfindungsgemäßen Verfahren herstellbar ist. Die oben für das erfindungsgemäße Verfahren als bevorzugt, besonders bevorzugt und ganz besonders bevorzugt herausgestellten Merkmale beziehen sich ebenfalls auf das nach diesem Verfahren herstellbare cis-angereichertes MDACH. Auch kann das cis-angereicherte MDACH weitere Isomere des Diamino-1-methylcyclohexans enthalten, vor allem vicinale Diamino-1-methylcyclohexan-Verbindungen, wie zum Beispiel 2,3-Diaminomethylcyclohexan und 3,4-Diaminomethylcyclohexan. Der Anteil der weiteren Isomere des Diamino-1-methylcyclohexan beträgt üblicherweise 0 bis 1 Gew.-%, insbesondere weniger als 0,5 Gew.-%, bezogen auf das cis-angereicherte MDACH.

Insbesondere betrifft die vorliegende Offenbarung cis-angereichertes MDACH, bei dem es sich um ein Gemisch handelt, das 0 bis 100 Gew.-% 2,4-MDACH und 0 bis 100 Gew.-% 2,6-MDACH, bezogen auf die im Gemisch enthaltende Gesamtmenge an MDACH, enthält sowie einen Anteil an cis-Isomeren von 55 bis 100 Gew.-%, bezogen auf die im

Gemisch enthaltende Gesamtmenge an MDACH, hat.
Besonders bevorzugt hat das cis-angereicherte MDACH einen Anteil an cis-Isomeren von 60 bis 99 und ganz besonders bevorzugt 70 bis 95 Gew.-%, jeweils bezogen auf die im Gemisch enthaltende Gesamtmenge an MDACH.
Bevorzugt besteht das cis-angereicherte MDACH zu mehr als 95, besonders bevorzugt mehr als 98 und ganz besonders bevorzugt 99 oder sogar 100 Gew.-% aus MDACH.
Darüber hinaus enthält das cis-angereicherte MDACH bevorzugt 5 bis 95 Gew.-% 2,4-MDACH und 5 bis 95 Gew.-% 2,6-MDACH sowie besonders bevorzugt 50 bis 100 Gew.-% 2,4-MDACH und 0 bis 50 Gew.-% 2,6-MDACH, jeweils bezogen auf die im Gemisch enthaltende Gesamtmenge an MDACH.
Das cis-angereicherte MDACH enthält bevorzugt höchstens 10 und besonders bevorzugt weniger als 5 Gew.-% Hilfsstoff, bezogen auf die Gesamtmenge des cis-angereicherten MDACH. Ganz besonders bevorzugt ist das cis-angereicherte MDACH frei von Hilfsstoff. Hierunter ist zu verstehen, dass der Anteil an Hilfsstoff im cis-angereicherten MDACH weniger als 0,01 Gew.-%, bezogen auf die Gesamtmenge des cis-angereicherten MDACH, beträgt.
Alle MDACH-haltigen Gemische, insbesondere das MDACH-Ausgangsgemisch sowie trans- und cis-angereichertes MDACH werden mithilfe der Gaschromatographie (GC) untersucht. Hierzu wird das Gemisch in Dioxan gelöst. Diese Lösung wird mit Hilfe einer Spritze in den Gaschromatographen eingespritzt. Der Gaschromatograph ist mit einer 30 m langen Säule bestückt, die einen Innendurchmesser von 0,25 mm und einer Filmdicke von 0,5 $\mu$m aufweist. Die Säule selbst enthält als stationäre Phase 35 Gew.-% Diphenyl- und 65 Gew.-% Dimethylpolysiloxan (Säule RTX35-Amin der Firma Resteck Corporation). Als Trägergas oder bewegliche Phase wird Helium verwendet. Die Geschwindigkeit des Heliums wird mit 40ml/min eingestellt, so dass man mit einem eingestellten Split ratio (Teilungsverhältnis) von 40:1 einen konstanten Fluss von 1 mL/min He über die Säule hat. Der Gaschromatograph weist zur Bestimmung der zu untersuchenden Substanzen einen Flammenionisationsdetektor auf, der bei 280°C betrieben wird. Die Säule im Gaschromatographen wird bei einer Temperatur im Bereich von 100 bis 250°C betrieben.
Um die Flächen- und Gewichtsprozente der zu bestimmenden Peaks ermitteln zu können, wird dem in Dioxan gelösten Gemisch eine definierte Menge eines Standards (Dodecan) hinzugefügt. Die so erhaltene Mischung wird mit einer Einspritztemperatur von 100°C und einen Eingangsdruck von 1 bar in die Säule gespritzt. Zuerst wird eine Heizrate von 1°C/min eingestellt, die so lange beibehalten wird bis eine Temperatur für die Säule von 120°C erreicht ist. Sobald diese Temperatur erreicht ist wird die Heizrate der Säule auf 5°C/min umgestellt und bis zur Endtemperatur von 250°C aufrechterhalten. Anschließend wird die Säulentemperatur für 10 min bei 250°C gehalten.
Das erfindungsgemäße Verfahren wird in den nachfolgenden Beispielen näher erläutert. Die angegebenen Analysedaten basieren auf gaschromatographischer Analytik, so wie oben beschrieben.

Beispiele:

Sofern nichts anderes angegeben ist, wurde in allen Beispielen Baxxodur ECX210 als MDACH-Ausgangsgemisch eingesetzt.
Zusammensetzung Baxxodur ECX 210:
trans-Isomer: 36 bis 38 Gew.-%
2,4-MDACH: 80 bis 90 Gew.-%
2,6-MDACH: 10 bis 20 Gew.-%
Die Angaben beziehen sich jeweils auf die im MDACH-Ausgangsgemisch enthaltende Gesamtmenge an MDACH.
Das MDACH-Ausgangsgemisch bestand zu $\geq$ 98 Gew.-% aus MDACH.

Beispiel 1:

Batch-Destillation mit (erfindungsgemäß) und ohne (nicht erfindungsgemäß) Hilfsstoff

In einem 1,6 Liter Miniplantreaktor, versehen mit einem Ankerrührer und Thermostat, wurden 800 g Baxxodur ECX 210 (MDACH-Ausgangsgemisch) und der jeweilige Hilfsstoff unter Luftausschluss vorgelegt, wobei das Stoffmengenverhältnis des Hilfsstoffs und der Stoffmenge aller Isomere des 2,4-Diamino-1-methylcyclohexans und 2,6-Diamino-1-methylcyclohexans 1 betrug. Die Destillation erfolgte mithilfe einer Kolonne (Sulzer DX Packung, $N_{th}$ = 30-45) mit Rücklaufteiler bei ca. 20 mbar Kopfdruck und einem Rücklaufverhältnis von 1. Während der Destillation wurde eine Fraktion von 100 mL entnommen und anschließend mittels GC analysiert.

Tabelle 1: Anteil an trans-Isomeren in Abhängigkeit vom eingesetzten Hilfsstoff. Alle Angaben sind in Gew.-% und beziehen sich auf die im MDACH-Ausgangsgemisch beziehungsweise die in der Fraktion enthaltende Gesamtmenge an MDACH.

| Hilfsstoff | Fraktion |
|---|---|
| Ohne Hilfsstoff | 44,15 |
| Glycerin | 46,03 |
| 1,3-Propandiol | 49,29 |
| Triethanolamin | 46,01 |
| Triethylenglykol | 45,39 |
| Diglycerin | 45,03 |
| 1,4-Butandiol | 46,20 |
| N-(2-Hydroxyethyl)-anilin | 45,82 |
| cis-1,4-Butendiol | 47,22 |
| 1,5-Pentandiol | 47,09 |
| N-Methyldiethanolamin | 46,07 |
| 4-(2-Hydroxyethyl)morpholin | 46,86 |

Die Ergebnisse zeigen, dass der Einsatz eines Hilfsstoffs zur Verbesserung der Trenneffizienz bezüglich der trans-Isomere führt. 1,3-Propandiol weist die höchste Trenneffizienz auf.

Beispiel 2:

Batch-Destillation mit Sulfolan als Hilfsstoff (nicht erfindungsgemäß)

Als MDACH-Ausgangsgemisch wurde Baxxodur ECX 210 und als Hilfsstoff Sulfolan eingesetzt. Versuchsaufbau und Versuchsdurchführung entsprechen denen aus Beispiel 1, mit dem Unterscheid, dass der Kopfdruck während der Destillation ca. 50 mbar betrug. Während der Destillation wurde eine Fraktion von 100 mL entnommen und anschließend mittels GC analysiert.

| Hilfsstoff | Fraktion |
|---|---|
| Ohne Sulfolan | 46,61 |
| Mit Sulfolan | 45,90 |

Tabelle 2: Anteil an trans-Isomeren in Abhängigkeit vom Einsatz des Hilfsstoffs Sulfolan. Alle Angaben sind in Gew.-% und beziehen sich auf die im MDACH-Ausgangsgemisch beziehungsweise die in der Fraktion enthaltende Gesamtmenge an MDACH.

Die Ergebnisse zeigen, dass durch eine Zugabe von Sulfolan als Hilfsstoff keine signifikante Verbesserung der Destillationsausbeute an trans-Isomeren erreicht wird, sondern eine Verschlechterung im Vergleich zur Destillation ohne Sulfolan eintritt.

Beispiel 3:

Batch-Destillation mit 1,3-Propandiol bei unterschiedlicher Zusammensetzung des MDACH-Ausgangsgemischs (erfindungsgemäß).

Die Destillation erfolgte mithilfe einer Kolonne (Sulzer DX Packung+Montz A3-1000, $N_{th}$ = ca. 60) bei einem Kopfdruck von 50 mbar. Die gesamte Einsatzmenge an MDACH-Ausgangsgemisch und 1,3-Propandiol betrug ca. 1000-1200 g, wobei in Versuch a) Baxxodur ECX 210 und in Versuch b) ein Gemisch mit einem Anteil von ca. 44 Gew.-% an trans-Isomeren eingesetzt wurde. Das Stoffmengenverhältnis des 1,3-Propandiols (Hilfsstoff) und MDACH betrug

1. Das Rücklaufverhältnis wurde auf 2 eingestellt. Im Übrigen entsprachen Versuchsaufbau und Versuchsdurchführung denen aus Beispiel 1. Während der Destillation wurde eine Fraktion von 100 mL entnommen und anschließend mittels GC analysiert.

Tabelle 3: Anteil an trans-Isomeren in Abhängigkeit vom Anteil an trans-Isomeren im MDACH-Ausgangsgemisch. Alle Angaben sind in Gew.-% und beziehen sich auf die im MDACH-Ausgangsgemisch beziehungsweise die in der Fraktion enthaltende Gesamtmenge an MDACH.

| Versuch | Fraktion |
|---|---|
| a) | 52,66 |
| b) | 68,91 |

Beispiel 4:

Kontinuierliche Destillation mit 1,3-Propandiol als Hilfsstoff (erfindungsgemäß)

Die eingesetzten Kolonnen (Sulzer CY Packung, ca. $N_{th}$ = 45) sind jeweils mit einem Dünnschichtverdampfer und einem separaten Kondensator versehen. Zuläufe und Abzüge der Kolonne sind schematisch in Abbildung 1 für Beispiel 4.1 und Abbildung 2 für Beispiel 4.2 dargestellt. Als Hilfsstoff wurde 1,3-Propandiol und als MDACH-Ausgangsgemisch Baxxodur ECX 210 eingesetzt.

Beispiel 4.1:

1,3-Propandiol wurde im Verstärkerteil der Kolonne bei einem Druck von ca. 50 mbar eingespeist. Das MDACH-Ausgangsgemisch wurde im Abtriebsteil der Kolonne bei einem Druck von ca. 53 mbar eingespeist. Die Zuläufe waren flüssig-siedend.

Variante 1:

Zulaufmengen:

MDACH-Ausgangsgemisch = 1540 g/h,
1,3-Propandiol = 920 g/h

Abzüge:

Destillat = 300 g/h
Rücklauf: 2000 g/h

Ergebnisse (Variante 1):

Das als Destillat erhaltene trans-angereicherte MDACH hatte die folgende Zusammensetzung:

trans-Isomer: ca. 66 Gew.-%
2,4-MDACH: ca. 68 bis 69 Gew.-%
2,6-MDACH: ca. 31 bis 32 Gew.-%

Die Angaben beziehen sich jeweils auf die im Destillat enthaltende Gesamtmenge an MDACH.

MCHA war zu ca. 1 GC-Fl-% (GC-Flächenprozent) im Destillat enthalten. Das 1,3-Propandiol war nahezu vollständig im Sumpf enthalten.

Variante 2:

Zulaufmengen:

MDACH-Ausgangsgemisch = 1540 g/h,
1,3-Propandiol = 1250 g/h

Abzüge:

Destillat = 300 g/h
Rücklauf: 1700 bis 1800 g/h

Ergebnisse (Variante 2):

Das als Destillat erhaltene trans-angereicherte MDACH hatte die folgende Zusammensetzung:

trans-Isomer: ca. 74 Gew.-%
2,4-MDACH: ca. 64 Gew.-%
2,6-MDACH: ca. 36 Gew.-%

Die Angaben beziehen sich jeweils auf die im Destillat enthaltende Gesamtmenge an MDACH.
MCHA war zu ca. 1 GC-Fl.-% im Destillat enthalten. Der Anteil an1,3-Propandiol im Destillat betrug ca. 13,8 Gew.-%, bezogen auf die Gesamtmenge des Destillats.

Beispiel 4.2

Zuläufe und Abzüge der Kolonnen sind schematisch in Abbildung 2 dargestellt.

Der erste Destillationsschritt (DESTILLATON 1) entspricht der Variante 2 aus Beispiel 4.1. Die Zulaufzusammensetzung für die DESTILLATION 2 entspricht also der Zusammensetzung des in Variante 2 des Beispiels 4.1 erhaltenen Destillats.
Die folgenden Angaben bezüglich Variante 1 und 2 beziehen sich auf den zweiten Destillationsschritt (DESTILLATION 2).

Variante 1

Zulaufmenge: 1000 g/h

Abzüge:

Seitenstrom = 590 g/h,
Destillat (am Kopfkondensator) = 0 g/h,
Nachkondensator = ca. 10 bis 30 g/h
Rücklauf (Kolonne) = 2000 g/h

Temperaturen:

Kopf-Kondensator = 85 °C,
Nachkondensator = ca.15 °C

Ergebnisse (Variante 1):

Das im Seitenstrom erhaltene trans-angereicherte MDACH hatte die folgende Zusammensetzung:

trans-Isomer: ca. 74 Gew.-%
2,4-MDACH: ca. 60 Gew.-%
2,6-MDACH: ca. 40 Gew.-%

Die Angaben beziehen sich jeweils auf die im Seitenstrom enthaltende Gesamtmenge an MDACH.
Das 1,3-Propandiol war nahezu vollständig im Sumpf enthalten. Der MCHA-Anteil im Seitenstrom wurde durch den Nachkondensator am Kopf der Kolonne von ca. 1 GC-Fl.-% in der Zulaufzusammensetzung auf ca. 0,61-0,66 GC-Fl.-% verringert.

Variante 2:

Zulaufmenge: 1000 g/h

Abzüge:

Seitenstrom = 490 g/h,
Destillat (am Kopfkondensator) = 100 g/h,
Nachkondensator = ca. 10-30 g/h
Rücklauf (Kolonne) = 2000 g/h

Temperaturen:

Kopf-Kondensator = 90 °C,
Nachkondensator = ca. 15 °C

Ergebnisse (Variante 2):

Das im Seitenstrom erhaltene trans-angereicherte MDACH hatte die folgende Zusammensetzung:

trans-Isomer: ca. 74 Gew.-%
2,4-MDACH: ca. 60 Gew.-&
2,6-MDACH: ca. 40 Gew.-%

Die Angaben beziehen sich jeweils auf die im Seitenstrom enthaltende Gesamtmenge an MDACH.
Das 1,3-Propandiol war nahezu vollständig im Sumpf enthalten. Der MCHA-Anteil im Seitenstrom wurde durch den Nachkondensator am Kopf der Kolonne sowie den Destillatabzug am Kopf-Kondensator von ca. 1% in der Zulaufzusammensetzung auf ca. 0,57 GC-Fl.-% verringert.

Herstellung von cis-angereichertem MDACH und Rückgewinnung des Hilfsstoffs (erfindungsgemäß):

Das Sumpfprodukt aus Beispiel 4.1, Variante 1 wurde in einer Destillationsblase, ausgestattet mit einer Kolonne (Sulzer DX Packung + Montz A3-1000, $N_{th}$ = ca. 60) vorgelegt und bei einem Kopfdruck von ca. 250 mbar unter Luftausschluss destilliert. Man erhielt ein Sumpfprodukt, das zu ca. 65 Gew.-% aus 1,3-Propandiol bestand. Dieses kann erneut bei der Destillation des MDACH-Ausgangsgemischs eingesetzt werden. Als Destillat erhielt man ein Gemisch, das die folgende Zusammensetzung aufwies:

cis-Isomer: ca. 83 Gew.-%
2,4-MDACH: ca. 92 Gew.-%
2,6-MDACH: ca. 8 Gew.-%

Die Angaben beziehen sich jeweils auf die im Destillat enthaltende Gesamtmenge an MDACH.

## Patentansprüche

1. Verfahren zur Herstellung von trans-angereichertem MDACH, **dadurch gekennzeichnet, dass** die Destillation eines MDACH-Ausgangsgemischs in Gegenwart eines Hilfsstoffs durchgeführt wird, wobei trans-angereichertes MDACH abdestilliert wird,
   und wobei

   - der Hilfsstoff eine organische Verbindung mit

     ◦ einer molaren Masse von 62 bis 500 g/mol,
     ◦ einem Siedepunkt, der mindestens 5 °C über dem Siedepunkt von cis,cis-2,6-Diamino-1-methylcyclohexan liegt, wobei sich die Siedepunkte jeweils auf einen Druck von 50 mbar beziehen, und
     ◦ 2 bis 4 funktionellen Gruppen, bei denen es sich jeweils unabhängig voneinander um eine Alkohol-, primäre, sekundäre, oder tertiäre Aminogruppe handelt,

ist,
• das MDACH-Ausgangsgemisch 0 bis 100 Gew.-% 2,4-Diamino-1-methylcyclohexan (2,4-MDACH) und 0 bis 100 Gew.-% 2,6-Diamino-1-methylcyclohexan (2,6-MDACH), bezogen auf die im MDACH-Ausgangsgemisch enthaltende Gesamtmenge an MDACH (= 2,4- und 2,6-MDACH), enthält und
wobei das MDACH-Ausgangsgemisch sowohl trans- als auch cis-Isomere enthält,
und
• trans-angereichertes MDACH ein Gemisch ist, das 0 bis 100 Gew.-% 2,4-MDACH und 0 bis 100 Gew.-% 2,6-MDACH, bezogen auf die im Gemisch enthaltende Gesamtmenge an MDACH, enthält, wobei der Anteil an trans-Isomeren im Gemisch, bezogen auf die im Gemisch enthaltene Gesamtmenge an MDACH, höher ist, als der Anteil an trans-Isomeren im MDACH-Ausgangsgemisch, bezogen auf die im MDACH-Ausgangsgemisch enthaltene Menge an MDACH.

**2.** Verfahren zur Herstellung von cis- und trans-angereichertem MDACH, das die folgenden Schritte umfasst:

I. Destillation eines MDACH-Ausgangsgemischs gemäß Anspruch 1,
II. Destillation des in Schritt I erhaltenen Sumpfprodukts, wobei cis-angereichertes MDACH abdestilliert wird,

wobei
cis-angereichertes MDACH ein Gemisch ist, das 0 bis 100 Gew.-% 2,4-MDACH und 0 bis 100 Gew.-% 2,6-MDACH, bezogen auf die im Gemisch enthaltende Gesamtmenge an MDACH, enthält, wobei der Anteil an cis-Isomeren im Gemisch, bezogen auf die im Gemisch enthaltene Gesamtmenge an MDACH, höher ist, als der Anteil an cis-Isomeren im MDACH-Ausgangsgemisch, bezogen auf die MDACH-Ausgangsgemisch enthaltene Menge an MDACH.

**3.** Verfahren nach Anspruch 1, **dadurch gekennzeichnet, dass** es kontinuierlich durchgeführt wird und die folgenden Schritte umfasst:

a) Zuführen des Hilfsstoffs in eine Kolonne (K),
b) Zuführen des MDACH-Ausgangsgemischs in K,
c) Abdestillieren von trans-angereichertem MDACH aus K.

**4.** Verfahren nach Anspruch 2, **dadurch gekennzeichnet, dass** es kontinuierlich wie folgt durchgeführt wird:

In Schritt I:

Destillation gemäß Anspruch 3,
In Schritt II:

a) Zuführen des Sumpfprodukts aus K in eine zweite Kolonne (K2),
b) Abdestillieren von cis-angereichertem MDACH aus K2.

**5.** Verfahren nach dem vorhergehenden Anspruch, **dadurch gekennzeichnet, dass** in einem Schritt III das Sumpfprodukt aus K2 in K rückgeführt wird.

**6.** Verfahren nach einem der Ansprüche 3 bis 5, **dadurch gekennzeichnet, dass** die Zuführung des Hilfsstoffs oberhalb der Zuführung des MDACH-Ausgangsgemischs erfolgt.

**7.** Verfahren nach einem der vorhergehenden Ansprüche, **dadurch gekennzeichnet, dass** die Destillation des MDACH-Ausgangsgemischs in Gegenwart des Hilfsstoffs bei einer Kopftemperatur von 80 bis 180 °C und einem Kopfdruck von 10 bis 500 mbar durchgeführt wird.

**8.** Verfahren nach einem der vorhergehenden Ansprüche, **dadurch gekennzeichnet, dass** der Hilfsstoff entweder

• 2 bis 4 Alkoholgruppen,
• eine primäre, sekundäre, oder tertiäre Aminogruppe und 1 bis 3 Alkoholgruppe(n), oder
• 2 bis 4 funktionellen Gruppen, bei denen es sich jeweils unabhängig voneinander um eine primäre, sekundäre, oder tertiäre Aminogruppe handelt,

besitzt.

9. Verfahren nach einem der vorhergehenden Ansprüche, **dadurch gekennzeichnet, dass** der Hilfsstoff neben den funktionellen Gruppen keine weiteren Heteroatome oder 1 bis 3 Ethergruppe(n) und ansonsten, neben den funktionellen Gruppen, keine weiteren Heteroatome besitzt.

10. Verfahren nach einem der vorhergehenden Ansprüche, **dadurch gekennzeichnet, dass** der Hilfsstoff eine molare Masse von 76 bis 300 g/mol besitzt.

11. Verfahren nach einem der Ansprüche 1 bis 7, **dadurch gekennzeichnet, dass** es sich bei dem Hilfsstoff um eine organische Verbindung handelt, mit

◦ einer molaren Masse von 62 bis 250 g/mol
◦ einem Siedepunkt, der mindestens 5 °C über dem Siedepunkt von cis,cis-2,6-Diamino-1-methylcyclohexan liegt, wobei sich die Siedepunkte jeweils auf einen Druck von 50 mbar beziehen,
◦ einem Schmelzpunkt von weniger als 60 °C bei einem Druck von 1 bar, und
◦ 2 bis 4 Alkohlgruppen,

wobei der Hilfsstoff neben den Alkohlgruppen keine weiteren Heteroatme oder eine oder zwei Ethergruppen und ansonsten, neben den Alkoholgruppen, keine weiteren Heteroatome besitzt.

12. Verfahren nach einem der Ansprüche 1 bis 7, **dadurch gekennzeichnet, dass** es sich bei dem Hilfsstoff um eine organische Verbindung handelt, mit

◦ einer molaren Masse von 75 bis 300 g/mol
◦ einem Siedepunkt, der mindestens 5 °C über dem Siedepunkt von cis,cis-2,6-Diamino-1-methylcyclohexan liegt, wobei sich die Siedepunkte jeweils auf einen Druck von 50 mbar beziehen,
◦ einem Schmelzpunkt von weniger als 60 °C bei einem Druck von 1 bar, und
◦ einer primäre, sekundären, oder tertiären Aminogruppe und 1 bis 3 Alkoholgruppe(n),

wobei der Hilfsstoff neben den funktionellen Gruppen keine weiteren Heteroatme oder eine Ethergruppe und ansonsten, neben den funktionellen Gruppen, keine weiteren Heteroatome besitzt.

13. Verfahren nach einem der Ansprüche 1 bis 7, **dadurch gekennzeichnet, dass** es sich bei dem Hilfsstoff um eine organische Verbindung handelt, mit

◦ einer molaren Masse von 100 bis 300 g/mol
◦ einem Siedepunkt, der mindestens 5 °C über dem Siedepunkt von cis,cis-2,6-Diamino-1-methylcyclohexan liegt, wobei sich die Siedepunkte jeweils auf einen Druck von 50 mbar beziehen,
◦ einem Schmelzpunkt von weniger als 60 °C bei einem Druck von 1 bar, und
◦ 2 bis 4 funktionellen Gruppen, bei denen es sich jeweils unabhängig voneinander um eine primäre, sekundäre, oder tertiäre Aminogruppe handelt,

wobei der Hilfsstoff neben den funktionellen Gruppen keine weiteren Heteroatme besitzt.

14. Verfahren nach einem der Ansprüche 1 bis 7, **dadurch gekennzeichnet, dass** es sich bei dem Hilfsstoff um eine der folgenden Verbindungen handelt:

2-Methylpropan-1,3-diol, 1,2-Hexandiol, cis-1,2-Cyclopentandiol, trans-1,2-Cyclopentandiol, 1,3-Propandiol, 1,5-Pentandiol, 2-Methyl-1,3-propandiol, 1,3-Hexandiol, 2,4-Hexandiol, 1,3-Cyclobutandiol, 1,3-Cyclopentandiol, 1,3-Cyclohexandiol, cis- und trans-1,4-Butendiol, 1,4-Butandiol, 2,3-Dimethyl-1,4-Butandiol, 2,2-Dimethyl-1,4-Butandiol, 1,4-Pentandiol, 2,3-Dimethyl-1,5-pentandiol, 1,4-Hexandiol, 1,3,6-Hexantriol, 1,2,6-Hexantriol, Glycerin, Diglycerin, Diethylenglykol, Triethylenglycol, Dipropylenglycol, Diethanolamin, N-Methyldiethanolamin, N-Propyldiethanolamin, N-Butyldiethanolamin, Triethanolamin, N-Ethylpropanolamin, N-Propylethanolamin, N,N-Dipropylethanolamin, N,N-Dibutylethanolamin, Dipropanolamin, N-Methyldipropanolamin, N-Propyldipropanolamin, N-Butyldipropanolamin, Tripropanolamin, , Diisopropanolamin, N-Methyldiisopropanolamin, Triisopropanolamin, N-2-Methylaminopropanol, 4-(2-Hydroxyethyl)morpholin, Pentanolamin, Hydroxyethylpiperazin, N-(2-Hydroxyethyl)anilin, N,N-Di-(2-hydroxyethyl)anilin, 3-(Cyclohexylamino)proplyamin, Dipropylentriamin, Triethylentetramin, 3-(2-aminoethylamino)propylamin, Isophorondiamin.

**15.** Verfahren nach einem der Ansprüche 1 bis 7, **dadurch gekennzeichnet, dass** es sich bei dem Hilfsstoff um eine der folgenden Verbindungen handelt:

Glycerin, 1,3-Propandiol, 1,4-Butandiol, cis-1,4-Butendiol, Triethylenglykol, Diglycerin, 3-(Cyclohexylamino)propylamin, 4-(2-Hydroxyethyl)morpholin, N-(2-Hydroxyethyl)-aniln, Triethanolamin, N-Methyldiethanolamin.

**16.** Verfahren nach einem der vorhergehenden Ansprüche, **dadurch gekennzeichnet, dass** das MDACH-Ausgangsgemisch zu mehr als 95 Gew.-% aus MDACH besteht.

**17.** Verfahren nach einem der vorhergehenden Ansprüche, **dadurch gekennzeichnet, dass** das MDACH-Ausgangsgemisch,

5 bis 95 Gew.-% 2,4-MDACH und
5 bis 95 Gew.-% 2,6-MDACH,

bezogen auf die im MDACH-Ausgangsgemisch enthaltende Gesamtmenge an MDACH, enthält.

**18.** Verfahren nach einem der vorhergehenden Ansprüche, **dadurch gekennzeichnet, dass** das MDACH-Ausgangsgemisch einen Anteil an trans-Isomeren von 5 bis 60 Gew.-% und einen Anteil an cis-Isomeren von 40 bis 95 Gew.-%, bezogen auf die im MDACH-Ausgangsgemisch enthaltende Gesamtmenge an MDACH, hat.

**19.** Verfahren nach einem der vorhergehenden Ansprüche, **dadurch gekennzeichnet, dass** das trans-angereicherte MDACH einen Anteil an trans-Isomeren von 55 bis 100 Gew.-%, bezogen auf die im Gemisch enthaltende Gesamtmenge an MDACH, hat.

**20.** Verfahren nach einem der vorhergehenden Ansprüche, **dadurch gekennzeichnet, dass** das trans-angereicherte MDACH zu mehr als 95 Gew.-% aus MDACH besteht.

**21.** Verfahren nach einem der vorhergehenden Ansprüche, **dadurch gekennzeichnet, dass** das trans-angereicherte MDACH

5 bis 95 Gew.-% 2,4-MDACH und
5 bis 95 Gew.-% 2,6-MDACH,

bezogen auf die im Gemisch enthaltende Gesamtmenge an MDACH, enthält.

**22.** Verfahren nach einem der Ansprüche 2 bis 21, **dadurch gekennzeichnet, dass** das cis-angereicherte MDACH eine Anteil an cis-Isomeren von 55 bis 100 Gew.-%, bezogen auf die im Gemisch enthaltende Gesamtmenge an MDACH, hat.

**23.** Verfahren nach einem der Ansprüche 2 bis 22, **dadurch gekennzeichnet, dass** das cis-angereicherte MDACH zu mehr als 95 Gew.-% aus MDACH besteht.

**24.** Verfahren nach einem der Ansprüche 2 bis 23, **dadurch gekennzeichnet, dass** das cis-angereicherte MDACH

5 bis 95 Gew.-% 2,4-MDACH und
5 bis 95 Gew.-% 2,6-MDACH,

bezogen auf die im Gemisch enthaltende Gesamtmenge an MDACH, enthält.

## Claims

**1.** A process for preparing trans-enriched MDACH, which comprises conducting the distillation of an MDACH starting mixture in the presence of an auxiliary, wherein trans-enriched MDACH is distilled off,
and wherein

• the auxiliary is an organic compound having

◦ a molar mass of 62 to 500 g/mol,
◦ a boiling point at least 5°C above the boiling point of cis,cis-2,6-diamino-1-methylcyclohexane, where the boiling points are each based on a pressure of 50 mbar, and
◦ 2 to 4 functional groups, each of which is independently an alcohol group or a primary, secondary or tertiary amino group,

• the MDACH starting mixture comprises 0% to 100% by weight of 2,4-diamino-l-methylcyclohexane (2,4-MDACH) and 0% to 100% by weight of 2,6-diamino-l-methylcyclohexane (2,6-MDACH), based on the total amount of MDACH (= 2,4- and 2,6-MDACH) present in the MDACH starting mixture, and wherein the MDACH starting mixture comprises both trans and cis isomers,
and
• trans-enriched MDACH is a mixture comprising 0% to 100% by weight of 2,4-MDACH and 0% to 100% by weight of 2,6-MDACH, based on the total amount of MDACH present in the mixture, where the proportion of trans isomers in the mixture, based on the total amount of MDACH present in the mixture, is higher than the proportion of trans isomers in the MDACH starting mixture, based on the amount of MDACH present in the MDACH starting mixture.

**2.** A process for preparing cis- and trans-enriched MDACH, comprising the following steps:

I. distilling an MDACH starting mixture according to claim 1,
II. distilling the bottom product obtained in step I, with distillative removal of cis-enriched MDACH,

wherein
cis-enriched MDACH is a mixture comprising 0% to 100% by weight of 2,4-MDACH and 0% to 100% by weight of 2,6-MDACH, based on the total amount of MDACH present in the mixture, where the proportion of cis isomers in the mixture, based on the total amount of MDACH present in the mixture, is higher than the proportion of cis isomers in the MDACH starting mixture, based on the amount of MDACH present in the MDACH starting mixture.

**3.** The process according to claim 1, which is performed continuously and comprises the following steps:

a) feeding the auxiliary into a column (K),
b) feeding the MDACH starting mixture into K,
c) distilling trans-enriched MDACH out of K.

**4.** The process according to claim 2, which is performed continuously as follows:

In step I:

distillation according to claim 3,
In step II:

a) feeding the bottom product from K into a second column (K2),
b) distilling cis-enriched MDACH out of K2.

**5.** The process according to the preceding claim, wherein the bottom product from K2 is recycled into K in a step III.

**6.** The process according to any of claims 3 to 5, wherein the auxiliary is fed in above the feed of the MDACH starting mixture.

**7.** The process according to any of the preceding claims, wherein the distillation of the MDACH starting mixture in the presence of the auxiliary is conducted at a top temperature of 80 to 180°C and a top pressure of 10 to 500 mbar.

**8.** The process according to any of the preceding claims, wherein the auxiliary has either

• 2 to 4 alcohol groups,
• a primary, secondary or tertiary amino group and 1 to 3 alcohol group(s), or

• 2 to 4 functional groups, each of which is independently a primary, secondary or tertiary amino group.

**9.** The process according to any of the preceding claims, wherein the auxiliary, aside from the functional groups, has no further heteroatoms or 1 to 3 ether group(s) and otherwise, aside from the functional groups, has no further heteroatoms.

**10.** The process according to any of the preceding claims, wherein the auxiliary has a molar mass from 76 to 300 g/mol.

**11.** The process according to any of claims 1 to 7, **characterized in that** the auxiliary is an organic compound having

◦ a molar mass of 62 to 250 g/mol
◦ a boiling point at least 5°C above the boiling point of cis, cis-2,6-diamino-1-methylcyclohexane, where the boiling points are each based on a pressure of 50 mbar,
◦ a melting point of less than 60°C at a pressure of 1 bar, and
◦ 2 to 4 alcohol groups,

where the auxiliary, aside from the alcohol groups, has no further heteroatoms or one or two ether groups and otherwise, aside from the alcohol groups, has no further heteroatoms.

**12.** The process according to any of claims 1 to 7, **characterized in that** the auxiliary is an organic compound having

◦ a molar mass of 75 to 300 g/mol
◦ a boiling point at least 5°C above the boiling point of cis, cis-2,6-diamino-1-methylcyclohexane, where the boiling points are each based on a pressure of 50 mbar,
◦ a melting point of less than 60°C at a pressure of 1 bar, and
◦ a primary, secondary or tertiary amino group and 1 to 3 alcohol group(s),

where the auxiliary, aside from the functional groups, has no further heteroatoms or one ether group and otherwise, aside from the functional groups, has no further heteroatoms.

**13.** The process according to any of claims 1 to 7, **characterized in that** the auxiliary is an organic compound having

◦ a molar mass of 100 to 300 g/mol
◦ a boiling point at least 5°C above the boiling point of cis, cis-2,6-diamino-1-methylcyclohexane, where the boiling points are each based on a pressure of 50 mbar,
◦ a melting point of less than 60°C at a pressure of 1 bar, and
◦ 2 to 4 functional groups, which are each independently a primary, secondary or tertiary amino group,

where the auxiliary, aside from the functional groups, has no further heteroatoms.

**14.** The process according to any of claims 1 to 7, wherein the auxiliary is one of the following compounds:

2-methylpropane-1,3-diol, hexane-1,2-diol, cis-cyclopentane-1,2-diol, trans-cyclopentane-1,2-diol, propane-1,3-diol, pentane-1,5-diol, 2-methylpropane-1,3-diol, hexane-1,3-diol, hexane-2,4-diol, cyclobutane-1,3-diol, cyclopentane-1,3-diol, cyclohexane-1,3-diol, cis- and trans-butene-1,4-diol, butane-1,4-diol, 2,3-dimethylbutane-1,4-diol, 2,2-dimethylbutane-1,4-diol, pentane-1,4-diol, 2,3-dimethylpentane-1,5-diol, hexane-1,4-diol, hexane-1,3,6-triol, hexane-1,2,6-triol, glycerol, diglycerol, diethylene glycol, triethylene glycol, dipropylene glycol, diethanolamine, N-methyldiethanolamine, N-propyldiethanolamine, N-butyldiethanolamine, triethanolamine, N-ethylpropanolamine, N-propylethanolamine, N,N-dipropylethanolamine, N,N-dibutylethanolamine, dipropanolamine, N-methyldipropanolamine, N-propyldipropanolamine, N-butyldipropanolamine, tripropanolamine, diisopropanolamine, N-methyldiisopropanolamine, triisopropanolamine, N-2-methylaminopropanol, 4-(2-hydroxyethyl)morpholine, pentanolamine, hydroxyethylpiperazine, N-(2-hydroxyethyl)aniline, N,N-di(2-hydroxyethyl)aniline, 3-(cyclohexylamino)propylamine, dipropylenetriamine, triethylenetetramine, 3-(2-aminoethylamino)propylamine, isophoronediamine.

**15.** The process according to any of claims 1 to 7, wherein the auxiliary is one of the following compounds:

glycerol, propane-1,3-diol, butane-1,4-diol, cis-butene-1,4-diol, triethylene glycol, diglycerol, 3-(cyclohexylamino)propylamine, 4-(2-hydroxyethyl)morpholine, N-(2-hydroxyethyl)aniline, triethanolamine, N-methyldiethanolamine.

**16.** The process according to any of the preceding claims, wherein the MDACH starting mixture consists of MDACH to an extent of more than 95% by weight.

**17.** The process according to any of the preceding claims, wherein the MDACH starting mixture comprises
5% to 95% by weight of 2,4-MDACH and
5% to 95% by weight of 2,6-MDACH,
based on the total amount of MDACH present in the MDACH starting mixture.

**18.** The process according to any of the preceding claims, wherein the MDACH starting mixture has a proportion of trans isomers of 5% to 60% by weight and a proportion of cis isomers of 40% to 95% by weight, based on the total amount of MDACH present in the MDACH starting mixture.

**19.** The process according to any of the preceding claims, wherein the trans-enriched MDACH has a proportion of trans isomers of 55% to 100% by weight, based on the total amount of MDACH present in the mixture.

**20.** The process according to any of the preceding claims, wherein the trans-enriched MDACH consists to an extent of more than 95% by weight of MDACH.

**21.** The process according to any of the preceding claims, wherein the trans-enriched MDACH comprises
5% to 95% by weight of 2,4-MDACH and
5% to 95% by weight of 2,6-MDACH,
based on the total amount of MDACH present in the mixture.

**22.** The process according to any of claims 2 to 21, wherein the cis-enriched MDACH has a proportion of cis isomers of 55% to 100% by weight, based on the total amount of MDACH present in the mixture.

**23.** The process according to any of claims 2 to 22, wherein the cis-enriched MDACH consists to an extent of more than 95% by weight of MDACH.

**24.** The process according to any of claims 2 to 23, wherein the cis-enriched MDACH comprises
5% to 95% by weight of 2,4-MDACH and
5% to 95% by weight of 2,6-MDACH,
based on the total amount of MDACH present in the mixture.

## Revendications

**1.** Procédé pour la préparation de MDACH enrichi en forme trans, **caractérisé en ce que** la distillation d'un mélange de départ à base de MDACH est réalisée en présence d'un adjuvant, du MDACH enrichi en forme trans étant éliminé par distillation et

- l'adjuvant étant un composé organique présentant
  - une masse molaire de 62 à 500 g/mole,
  - un point d'ébullition qui se situe au moins 5°C au-dessus du point d'ébullition du cis,cis-2,6-diamino-1-méthylcyclohexane, les points d'ébullition se référant chacun à une pression de 50 mbars et
  - 2 à 4 groupes fonctionnels, pour lesquels il s'agit à chaque fois, indépendamment les uns des autres, d'un groupe alcool ou d'un groupe amino primaire, secondaire ou tertiaire,
- le mélange de départ à base de MDACH contenant 0 à 100% en poids de 2,4-diamino-1-méthylcyclohexane (2,4-MDACH) et 0 à 100% en poids de 2,6-diamino-1-méthylcyclohexane (2,6-MDACH), par rapport à la quantité totale de MDACH (= 2,4-MDACH et 2,6-MDACH) contenue dans le mélange de départ à base de MDACH et le mélange de départ à base de MDACH contenant tant des isomères trans que des isomères cis et
- le MDACH enrichi en forme trans étant un mélange qui contient 0 à 100% en poids de 2,4-MDACH et 0 à 100% en poids de 2,6-MDACH, par rapport à la quantité totale de MDACH contenue dans le mélange, la

proportion d'isomères trans dans le mélange, par rapport à la quantité totale de MDACH contenue dans le mélange, étant supérieure à la proportion d'isomères trans dans le mélange de départ à base de MDACH, par rapport à la quantité de MDACH contenue dans le mélange de départ à base de MDACH.

**2.** Procédé pour la préparation de MDACH enrichi en forme cis et en forme trans, qui comprend les étapes suivantes :

I. distillation d'un mélange de départ à base de MDACH selon la revendication 1,
II. distillation du produit de fond obtenu dans l'étape I, le MDACH enrichi en forme cis étant éliminé par distillation,

le MDACH enrichi en forme cis étant un mélange qui contient 0 à 100% en poids de 2,4-MDACH et 0 à 100% en poids de 2,6-MDACH, par rapport à la quantité totale de MDACH contenue dans le mélange, la proportion d'isomères cis dans le mélange, par rapport à la quantité totale de MDACH contenue dans le mélange, étant supérieure à la proportion d'isomères cis dans le mélange de départ à base de MDACH, par rapport à la quantité de MDACH contenue dans le mélange de départ à base de MDACH.

**3.** Procédé selon la revendication 1, **caractérisé en ce qu'**il est réalisé en continu et comprend les étapes suivantes :

a) introduction de l'adjuvant dans une colonne (K),
b) introduction du mélange de départ à base de MDACH dans K,
c) élimination par distillation du MDACH enrichi en forme trans à partir de K.

**4.** Procédé selon la revendication 2, **caractérisé en ce qu'**il est réalisé en continu comme suit :

dans l'étape I :

distillation selon la revendication 3,

dans l'étape II :

a) introduction du produit du fond provenant de K dans une deuxième colonne (K2),
b) élimination par distillation de MDACH enrichi en forme cis à partir de K2.

**5.** Procédé selon la revendication précédente, **caractérisé en ce que**, dans une étape III, le produit de fond provenant de K2 est recyclé dans K.

**6.** Procédé selon l'une quelconque des revendications 3 à 5, **caractérisé en ce que** l'introduction de l'adjuvant a lieu au-dessus de l'introduction du mélange de départ à base de MDACH.

**7.** Procédé selon l'une quelconque des revendications précédentes, **caractérisé en ce que** la distillation du mélange de départ à base de MDACH est réalisée en présence de l'adjuvant à une température de tête de 80 à 180°C et à une pression de tête de 10 à 500 mbars.

**8.** Procédé selon l'une quelconque des revendications précédentes, **caractérisé en ce que** l'adjuvant présente

- soit 2 à 4 groupes alcool,
- soit un groupe amino primaire, secondaire ou tertiaire et 1 à 3 groupes alcool,
- soit 2 à 4 groupes fonctionnels, pour lesquels il s'agit à chaque fois, indépendamment les uns des autres, d'un groupe amino primaire, secondaire ou tertiaire.

**9.** Procédé selon l'une quelconque des revendications précédentes, **caractérisé en ce que** l'adjuvant ne présente, outre les groupes fonctionnels, pas d'autres hétéroatomes ou présente 1 à 3 groupes éther et, pour le reste, outre les groupes fonctionnels, ne présente pas d'autres hétéroatomes.

**10.** Procédé selon l'une quelconque des revendications précédentes, **caractérisé en ce que** l'adjuvant présente une masse molaire de 76 à 300 g/mole.

**11.** Procédé selon l'une quelconque des revendications 1 à 7, **caractérisé en ce qu'**il s'agit, pour l'adjuvant, d'un composé organique présentant

- une masse molaire de 62 à 250 g/mole,
- un point d'ébullition qui se situe au moins 5°C au-dessus du point d'ébullition du cis,cis-2,6-diamino-1-méthylcyclohexane, les points d'ébullition se référant chacun à une pression de 50 mbars,
- un point de fusion inférieur à 60°C à une pression de 1 bar et
- 2 à 4 groupes alcool,

l'adjuvant ne présentant, outre les groupes alcool, pas d'autres hétéroatomes ou présentant un à deux groupes éther et, pour le reste, outre les groupes alcool, ne présentant pas d'autres hétéroatomes.

**12.** Procédé selon l'une quelconque des revendications 1 à 7, **caractérisé en ce qu'**il s'agit, pour l'adjuvant, d'un composé organique présentant

- une masse molaire de 75 à 300 g/mole,
- un point d'ébullition qui se situe au moins 5°C au-dessus du point d'ébullition du cis,cis-2,6-diamino-1-méthylcyclohexane, les points d'ébullition se référant chacun à une pression de 50 mbars,
- un point de fusion inférieur à 60°C à une pression de 1 bar et
- un groupe amino primaire, secondaire ou tertiaire et 1 à 3 groupes alcool,

l'adjuvant ne présentant, outre les groupes fonctionnels, pas d'autres hétéroatomes ou présentant un groupe éther et, pour le reste, outre les groupes fonctionnels, ne présentant pas d'autres hétéroatomes.

**13.** Procédé selon l'une quelconque des revendications 1 à 7, **caractérisé en ce qu'**il s'agit, pour l'adjuvant, d'un composé organique présentant

- une masse molaire de 100 à 300 g/mole,
- un point d'ébullition qui se situe au moins 5°C au-dessus du point d'ébullition du cis,cis-2,6-diamino-1-méthylcyclohexane, les points d'ébullition se référant chacun à une pression de 50 mbars,
- un point de fusion inférieur à 60°C à une pression de 1 bar et
- 2 à 4 groupes fonctionnels, pour lesquels il s'agit à chaque fois, indépendamment les uns des autres, d'un groupe amino primaire, secondaire ou tertiaire,

l'adjuvant ne présentant, outre les groupes fonctionnels, pas d'autres hétéroatomes.

**14.** Procédé selon l'une quelconque des revendications 1 à 7, **caractérisé en ce qu'**il s'agit, pour l'adjuvant, d'un composé des composés suivants :

2-méthylpropane-1,3-diol, 1,2-hexanediol, cis-1,2-cyclopentanediol, trans-1,2-cyclopentanediol, 1,3-propanediol, 1,5-pentanediol, 2-méthyl-1,3-propanediol, 1,3-hexanediol, 2,4-hexanediol, 1,3-cyclobutanediol, 1,3-cyclopentanediol, 1,3-cyclohexanediol, cis-1,4-butènediol et trans-1,4-butènediol, 1,4-butanediol, 2,3-diméthyl-1,4-butanediol, 2,2-diméthyl-1,4-butanediol, 1,4-pentanediol, 2,3-diméthyl-1,5-pentanediol, 1,4-hexanediol, 1,3,6-hexanetriol, 1,2,6-hexanetriol, glycérol, diglycérol, diéthylèneglycol, triéthylèneglycol, dipropylèneglycol, diéthanolamine, N-méthyldiéthanolamine, N-propyldiéthanolamine, N-butyldiéthanolamine, triéthanolamine, N-éthylpropanolamine, N-propyléthanolamine, N,N-dipropyléthanolamine, N,N-dibutyléthanolamine, dipropanolamine, N-méthyldipropanolamine, N-propyldipropanolamine, N-butyldipropanolamine, tripropanolamine, diisopropanolamine, N-méthyldiisopropanolamine, triisopropanolamine, N-2-méthylaminopropanol, 4-(2-hydroxyéthyl)morpholine, pentanolamine, hydroxyéthylpipérazine, N-(2-hydroxyéthyl)aniline, N,N-di-(2-hydroxyéthyl)aniline, 3-(cyclohexylamino)propylamine, dipropylènetriamine, triéthylènetétramine, 3-(2-aminoéthylamino)propylamine, isophoronediamine.

**15.** Procédé selon l'une quelconque des revendications 1 à 7, **caractérisé en ce qu'**il s'agit, pour l'adjuvant, d'un des composés suivants : glycérol, 1,3-propanediol, 1,4-butanediol, cis-1,4-butènediol, triéthylèneglycol, diglycérol, 3-(cyclohexylamino)propylamine, 4-(2-hydroxyéthyl)morpholine, N-(2-hydroxyéthyl)-aniline, triéthanolamine, N-méthyldiéthanolamine.

**16.** Procédé selon l'une quelconque des revendications précédentes, **caractérisé en ce que** le mélange de départ à base de MDACH est constitué, à raison de plus de 95% en poids, de MDACH.

**17.** Procédé selon l'une quelconque des revendications précédentes, **caractérisé en ce que** le mélange de départ à

base de MDACH contient 5 à 95% en poids de 2,4-MDACH et 5 à 95% en poids de 2,6-MDACH, par rapport à la quantité totale de MDACH contenue dans le mélange de départ à base de MDACH.

**18.** Procédé selon l'une quelconque des revendications précédentes, **caractérisé en ce que** le mélange de départ à base de MDACH contient une proportion d'isomères trans de 5 à 60% en poids et une proportion d'isomères cis de 40 à 95% en poids, par rapport à la quantité totale de MDACH contenue dans le mélange de départ à base de MDACH.

**19.** Procédé selon l'une quelconque des revendications précédentes, **caractérisé en ce que** le MDACH enrichi en forme trans présente une proportion d'isomères trans de 55 à 100% en poids, par rapport à la quantité totale de MDACH contenue dans le mélange.

**20.** Procédé selon l'une quelconque des revendications précédentes, **caractérisé en ce que** le MDACH enrichi en forme trans est constitué, à raison de plus de 95% en poids, de MDACH.

**21.** Procédé selon l'une quelconque des revendications précédentes, **caractérisé en ce que** le MDACH enrichi en forme trans contient 5 à 95% en poids de 2,4-MDACH et 5 à 95% en poids de 2, 6-MDACH, par rapport à la quantité totale de MDACH contenue dans le mélange.

**22.** Procédé selon l'une quelconque des revendications 2 à 21, **caractérisé en ce que** le MDACH enrichi en forme cis présente une proportion d'isomères cis de 55 à 100% en poids, par rapport à la quantité totale de MDACH contenue dans le mélange.

**23.** Procédé selon l'une quelconque des revendications 2 à 22, **caractérisé en ce que** le MDACH enrichi en forme cis est constitué, à raison de plus de 95% en poids, de MDACH.

**24.** Procédé selon l'une quelconque des revendications 2 à 23, **caractérisé en ce que** le MDACH enrichi en forme cis contient 5 à 95% en poids de 2, 4-MDACH et 5 à 95% en poids de 2,6-MDACH, par rapport à la quantité totale de MDACH contenue dans le mélange.

Abbildung 1 (DESTILLATION 1):

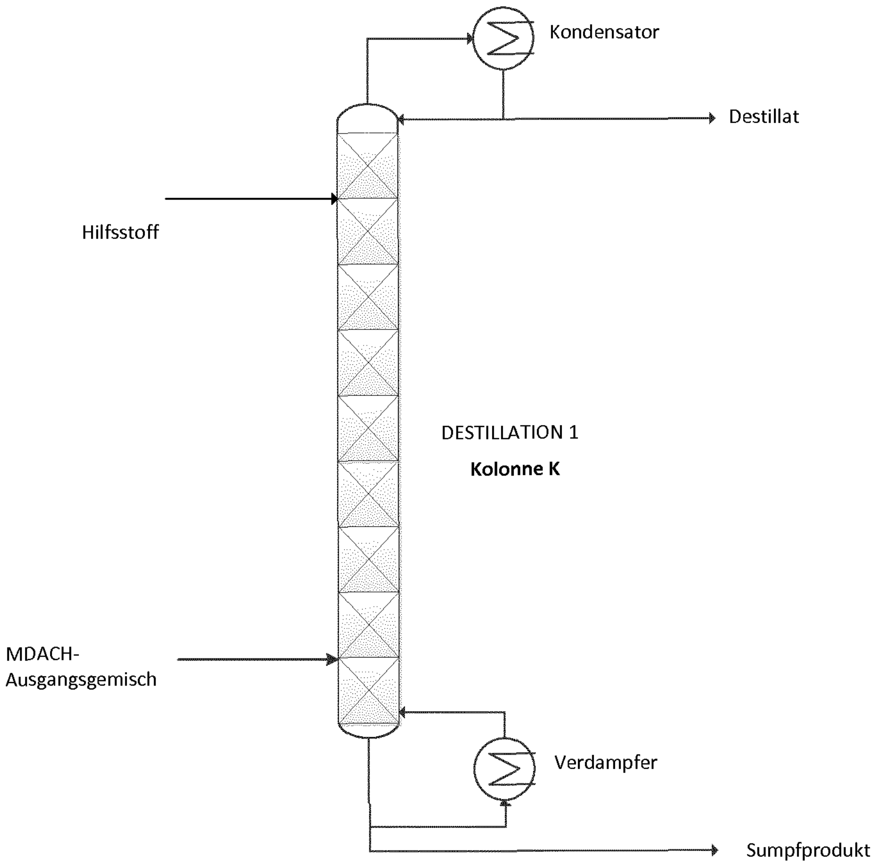

Abbildung 2 (DESTILLATION 1 und 2):

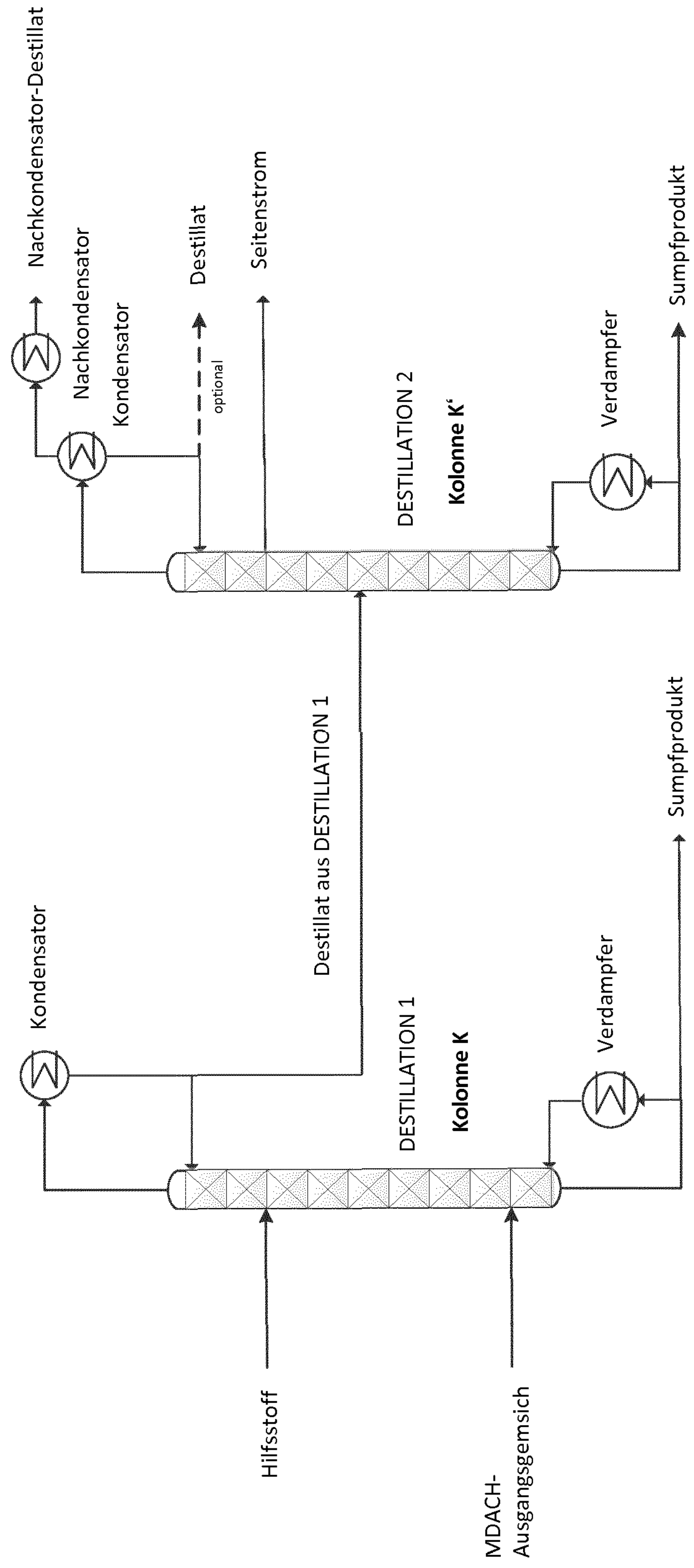

Abbildung 3 (Herstellung von cis-angereichertem MDACH und Rückgewinnung des Hilfsstoffs):

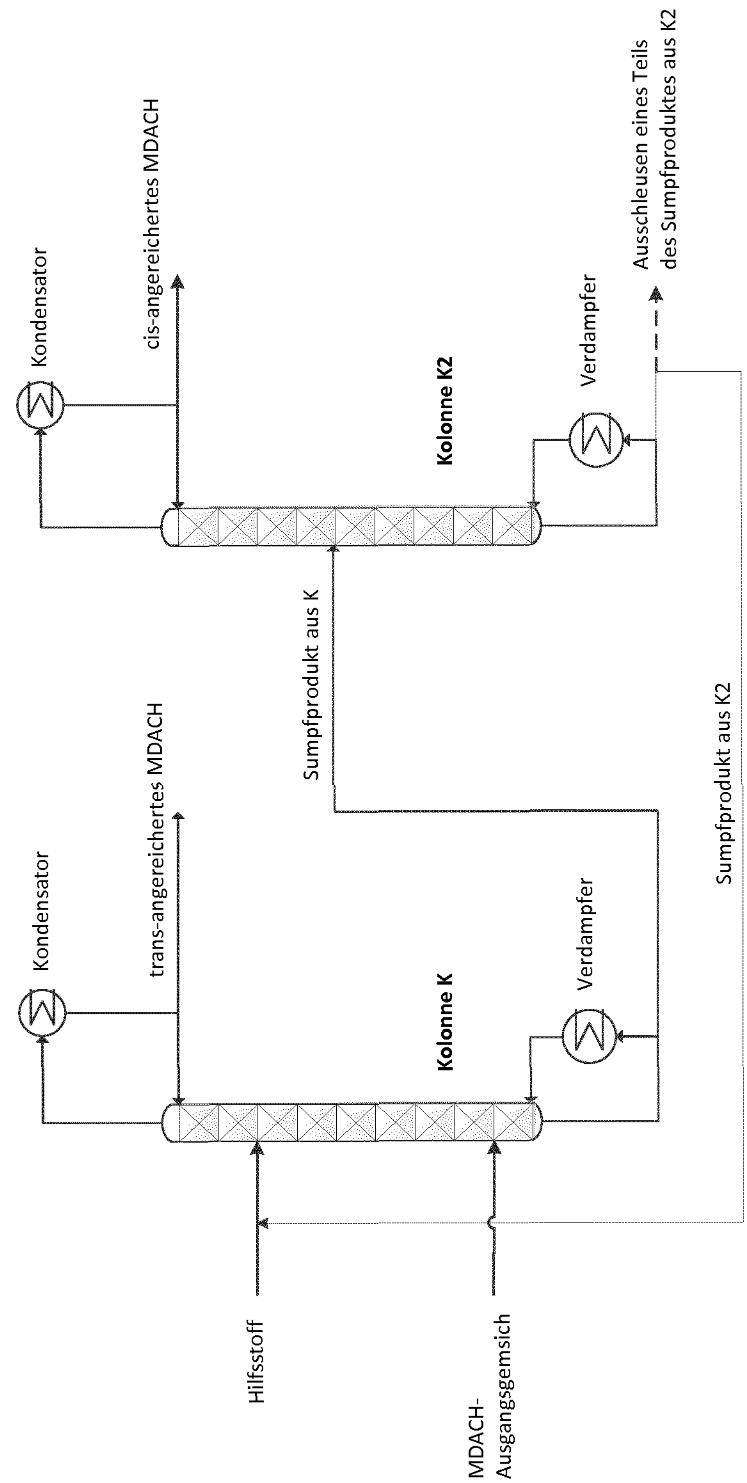

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- EP 0796839 A1
- WO 2011033104 A1